# EUROPÄISCHE PATENTSCHRIFT

(11) **EP 2 040 079 B1**
(45) Veröffentlichungstag und Bekanntmachung des Hinweises auf die Patenterteilung: **20.05.2009**
(21) Anmeldenummer: 07116749.8
(22) Anmeldetag: 19.09.2007
(51) Int. Cl.: G01N 35/00

(54) **Markierungsverfahren zur Ausschussmarkierung von Testelementen**
Marking method for marking rejects of test elements
Procédé de marquage destiné au marquage de déchet d'éléments test

(43) Veröffentlichungstag der Anmeldung: 25.03.2009
(73) Patentinhaber: F. Hoffmann-La Roche AG, 4070 Basel (CH); Roche Diagnostics GmbH, 68305 Mannheim (DE)
(72) Erfinder: Röper, Dr.Josef K., 67141 Neuhofen (DE); Frank, Martin, 67246 Dirmstein (DE); Schmidt, Günter, 68623 Lampertheim (DE); Finke, Dr.Werner, 64683 Einhausen (DE); Dick, Siegfried, 68307 Mannheim (DE); Stubenbord, Peter, 67141 Neuhofen (DE)
(74) Vertreter: Huhn, Michael

(56) Entgegenhaltungen:
- EP-A- 0 132 790
- EP-A- 1 826 705
- US-A1- 2004 048 359

## Beschreibung

### Gebiet der Erfindung

Die Erfindung betrifft ein Markierungsverfahren zur Markierung fehlerhafter Testelemente sowie ein Herstellungsverfahren zur Herstellung von Testelementen, welches ein erfindungsgemäßes Markierungsverfahren beinhaltet. Weiterhin betrifft die Erfindung eine Markierungsvorrichtung, insbesondere zur Durchführung eines erfindungsgemäßen Markierungsverfahrens, sowie eine Herstellungsvorrichtung zur Herstellung von Testelementen, welche eine erfindungsgemäße Markierungsvorrichtung umfasst. Weiterhin betrifft die Erfindung ein analytisches Testgerät, welches ein nach dem erfindungsgemäßen Herstellungsverfahren hergestelltes Testelement verwendet. Derartige Markierungs- und Herstellverfahren, Markierungs- und Herstellvorrichtungen sowie analytische Testgeräte werden insbesondere in der chemischen Analytik sowie in der Medizintechnik eingesetzt. Mittels der Testelemente können beispielsweise Analyte wie z.B. Metabolite in Proben, insbesondere in flüssigen Proben wie z.B. in Blut, Urin, interstitieller Flüssigkeit oder anderen Körperflüssigkeiten, qualitativ und/oder quantitativ nachgewiesen werden. Ein wesentliches Anwendungsbeispiel der vorliegenden Erfindung liegt im Bereich der Blutzuckerdiagnostik.

### Stand der Technik

In vielen Bereichen der Technik, der Naturwissenschaften und der Medizin müssen zuverlässig Analyte in Proben qualitativ und/oder quantitativ nachgewiesen werden. Dies erfolgt in vielen Fällen mit Testelementen, welche auf einen oder mehrere Analyte sensitiv reagieren. So können insbesondere Testelemente verwendet werden, welche mindestens ein Testmaterial umfassen, welches bei Anwesenheit des Analyten in der Probe bzw. bei Kontakt mit dem Analyten mindestens eine messbare Eigenschaft ändert. Bei diesen Eigenschaften kann es sich beispielsweise, wie unten näher ausgeführt, um elektrische und /oder optische Eigenschaften handeln.

Ein wesentliches Anwendungsgebiet der vorliegenden Erfindung, auf welches die Erfindung jedoch nicht beschränkt ist, ist die medizinische Diagnostik. So ist beispielsweise die Überwachung von Blutglukosekonzentrationen für Diabetiker ein essentieller Bestandteil des Tagesablaufs. Dabei muss die Blutglukosekonzentration schnell und einfach in der Regel mehrmals am Tag bestimmt werden, um gegebenenfalls entsprechende medizinische Maßnahmen ergreifen zu können. Um den Tagesablauf des Diabetikers nicht mehr als nötig einzuschränken, werden dabei häufig entsprechende mobile Geräte eingesetzt, welche einfach zu transportieren und zu handhaben sein sollten, so dass die Messung der Blutglukosekonzentration beispielsweise am Arbeitsplatz oder auch in der Freizeit schnell und einfach und dennoch zuverlässig erfolgen kann. Auch stationäre Geräte lassen sich jedoch einsetzen, wie beispielsweise Geräte, welche für Krankenhäuser, Arztpraxen oder Pflegeeinrichtungen konzipiert sind.

Derzeit sind verschiedene Analysegeräte am Markt, welche teilweise nach unterschiedlichen Messmethoden funktionieren. Dabei kommen verschiedene Diagnoseverfahren zum Einsatz, beispielsweise optische oder auch elektrochemische Messverfahren. Ein wesentliches Element dieser Messverfahren sind häufig die bereits erwähnten Testelemente, welche zumeist in Form von Teststreifen vorliegen. Beispielsweise kann es sich dabei um elektrochemische und/oder um optische Teststreifen handeln. Beispiele elektrochemischer Testelemente sind z. B. in US 5,286,362 beschrieben. Optische Testelemente sind beispielsweise in CA 2,050,677 offenbart. Auch andere Arten von Testelementen, beispielsweise implantierbare Testelemente (siehe z.B. EP 0 678 308 B1) sind bekannt und im Rahmen der vorliegenden Erfindung einsetzbar. Anstelle einzelner Testelemente, wie beispielsweise Teststreifen oder Teströhrchen, sind auch Testelemente bekannt, welche in einem Magazin oder in einer anderen Art von Lagervorrichtung aufgenommen sind. So können beispielsweise mehrere Testelemente untereinander starr verbunden sein, beispielsweise im Rahmen einer Testscheibe, auf welcher sich mehrere Testfelder befinden. Andere Arten von Mehrfach-Testelementen sind beispielsweise im Rahmen von Bandkassetten bekannt, bei welchen eine Vielzahl von Testelementen bzw. Testfeldern auf einem gemeinsamen Band angeordnet sind, so dass diese beispielsweise nacheinander verwendet werden können. Weitere Ausführungsformen von Magazinen sind Trommelmagazine, bei welchen mehrere Testelemente in einer Magazintrommel aufgenommen sind. Auch andere Ausführungsformen sind bekannt.

Die Zuverlässigkeit des Nachweises des Analyten spielt insbesondere bei quantitativen Nachweisverfahren in der medizinischen Diagnostik eine entscheidende Rolle. So hängt von dem Ergebnis des Nachweises in der Regel eine Reihe weiterer Entscheidungen ab, wie beispielsweise die Entscheidung über eine Insulin-Medikation oder die Entscheidung über eine anderweitige medizinische Behandlung. Insofern ist ein effizientes Qualitätsmanagement bei der Herstellung der Testelemente erforderlich, welches zuverlässig verhindert, dass fehlerhafte Testelemente in Umlauf geraten oder, sollten diese in Umlauf geraten sein, dort verwendet werden. Dieses Qualitätsmanagement kann eine Vielzahl von Testverfahren beinhalten, welche bereits während des Herstellungsprozesses oder nach der Herstellung die Testelemente bestimmten Funktionstests unterwerfen können. So können beispielsweise Tests durchgeführt werden, welche (z.B. mittels einer Bilderkennung, elektronischer Messungen, optischer Messungen oder Kombinationen von Messungen) bestimmte Funktionalitäten der Testelemente überprüfen und auf diese Weise mit einer gewissen Wahrscheinlichkeit fehlerhafte Testelemente erkennen.

Die Herstellung derartiger Testelemente ist in der Regel ein Massenprozess, bei welchem großtechnisch mit hohem Durchsatz eine Vielzahl von Testelementen produziert wird. Es ist daher in der Regel nicht möglich, bei Erkennen eines fehlerhaften Testelements dieses Testelement unmittelbar auszusondern. Aus dem Stand der Technik sind daher Verfahren bekannt, bei welchen als fehlerhaft erkannte Testelemente während oder nach dem Herstellungsverfahren als fehlerhaft markiert werden. Beispiele derartiger Markierungsverfahren sind in US 2004/0048359 A1 offenbart, wobei dort fehlerhafte Bereiche mittels eines Stiftes oder Markierers markiert werden. Ein anderes aus dem Stand der Technik bekanntes Verfahren ist in EP 0132790 A2 beschrieben. Hierbei wird eine Vielzahl von Testelementen auf einem Gesamtband hergestellt, und es wird nach Feststellung eines Fehlers eine geeignete Markierung in Form eines Farbpunktes oder einer magnetischen Markierung aufgebracht, um anschließend das fehlerhafte Testelement einfach und zuverlässig aussortieren zu können.

Die aus dem Stand der Technik bekannten Markierungs- und Herstellungsverfahren weisen jedoch in der Praxis zahlreiche Nachteile auf. So werden in der Regel bei den bekannten Markierungsverfahren zusätzliche Einsatzstoffe und Hilfsstoffe verwendet, wie beispielsweise Farben für Farbpunkte, Lacke, magnetische Materialien oder ähnliche Materialien. Diese zusätzliche Einsatz- und Hilfsstoffe können jedoch in Wechselwirkung mit der Funktionalität des Testelements treten und können beispielsweise die Funktionalität eines Testmaterials (z.B. einer Testchemie für den Nachweis von Blutglukose oder eines anderen Metaboliten) beeinflussen. So muss in der Regel eine Unbedenklichkeit der für die Markierung verwendeten Einsatz- und Hilfsstoffe ausführlich überprüft und belegt werden, um beispielsweise entsprechende behördliche Zulassungen zu erlangen.

Ein weiterer Nachteil bekannter Verfahren besteht darin, dass viele der bekannten Applikationsverfahren für die Einsatz- und Hilfsstoffe, welche zum Markieren verwendet werden, komplex und fehleranfällig sind. So können beispielsweise Lacke oder Farben für die Markierung mittels eines Druckverfahrens aufgebracht werden, welches jedoch für sich genommen in vielen Fällen fehlerträchtig ist.

Ein weiterer Nachteil besteht darin, dass die aufgebrachten Einsatz- und Hilfsstoffe, welche im Stand der Technik in der Regel für die Markierung verwendet werden, in vielen Fällen in flüssiger Form aufgebracht werden und daher nach dem Aufbringen eine Trocknungszeit erforderlich ist. Diese Trocknungszeiten der Markierung limitieren in vielen Fällen die Fertigungsgeschwindigkeit der Herstellungsprozesse und erhöhen somit die Herstellkosten beträchtlich.

### Aufgabe der Erfindung

Es ist daher eine Aufgabe der vorliegenden Erfindung, ein Markierungsverfahren bereitzustellen, welches die oben beschriebenen Nachteile bekannter Markierungsverfahren zumindest weitgehend vermeidet. Das Markierungsverfahren soll eine einfache, schnelle und zuverlässige Markierung fehlerhafter Testelemente ermöglichen, welche ohne eine nennenswerte Erhöhung der Herstellkosten auskommen soll.

### Beschreibung der Erfindung

Es wird ein Markierungsverfahren zur Markierung fehlerhafter Testelemente, ein Herstellungsverfahren, welches dieses Markierungsverfahren beinhaltet, eine Markierungsvorrichtung zur Markierung fehlerhafter Testelemente, eine Herstellungsvorrichtung zur Herstellung von Testelementen unter Einbeziehung einer Markierungsvorrichtung sowie ein analytisches Testgerät, welches nach dem erfindungsgemäßen Verfahren hergestellte Testelemente verwendet, vorgeschlagen. Vorteilhafte Weiterbildungen der Erfindung sind in den Unteransprüchen dargestellt. Diese Weiterbildungen können einzeln oder auch in Kombination miteinander verwirklicht sein. Der Wortlaut sämtlicher Ansprüche wird hiermit zum Inhalt der Beschreibung gemacht.

Die Testelemente sind eingerichtet, um mindestens einen Analyten in einer Probe nachzuweisen. Beispielsweise kann es sich dabei um Testelemente der oben beschriebenen, aus dem Stand der Technik bekannten Arten halten, beispielsweise Testelemente zum Nachweis von Metaboliten in flüssigen Proben, wie insbesondere in Blut, Urin, interstitiellem Fettgewebe oder anderen Körperflüssigkeiten. Auch für andere Arten von Analyten und Proben können jedoch Testelemente eingesetzt werden. Die Testelemente können beispielsweise in Streifenform, in Blättchenform, in Scheibenform, in Form von Bändern oder in ähnlichen Ausgestaltungen jeweils einzeln oder zu mehreren zusammengefasst ausgestaltet sein. Die Testelemente können für einen oder für mehrere Tests geeignet sein und können insbesondere ein oder mehrere Testfelder aufweisen, auf welche die Probe aufgebracht werden kann, bzw. mit denen die Probe in Kontakt gebracht werden kann.

Das Markierungsverfahren ist, wie bekannte Markierungsverfahren aus dem Stand der Technik, derart ausgestaltet, dass die Testelemente zumindest teilweise mit einer Fehlermarkierung versehen werden, welche eine Information über eine Fehlerhaftigkeit der Testelemente beinhaltet. Beispielsweise kann ein als fehlerhaft erkanntes Testelemente mit einer entsprechenden Fehlermarkierung versehen werden.

Im Gegensatz zum Stand der Technik, bei welchem beispielsweise eine Farbmarkierung erfolgt, umfassen die Testelemente mindestens ein strahlungssensitives Material. Zur Markierung werden die Testelemente mit mindestens einer Strahlung beaufschlagt, welche eingerichtet und/oder ausgewählt ist, um eine Markierung in Form mindestens einer optisch nachweisbaren Änderung in dem strahlungssensitiven Material zu bewirken.

Im Gegensatz zum Stand der Technik ist also kein Aufbringen eines zusätzlichen Markierungsstoffes auf das Testelement erforderlich, sondern es werden vorzugsweise strahlungssensitive Eigenschaften des Testelementes selbst ausgenutzt, um das Testelement zu markieren. Alternativ oder zusätzlich können jedoch auch von der Funktionalität der Testelemente, insbesondere von einer Testchemie in Testfeldern der Testelemente, unabhängige Markierungen auf die Testelemente aufgebracht werden, beispielsweise auf ein Trägerband der Testelemente oder auf separate, von den Testfeldern unabhängige Markierungsfelder. Die Markierung kann berührungslos erfolgen und beeinflusst daher eine Herstellung der Testelemente (z.B. in Form einer mechanischen Berührung) nicht. Dadurch lässt sich eine Beeinflussung wesentlicher Maschinenparameter, wie beispielsweise Zugkräfte auf ein Testelement-Band, vermeiden, wie sie beispielsweise bei der Beaufschlagung mit flüssigen Markierungsmitteln erfolgen kann. Zu dem ist das Aufbringen einer Ausschussmarkierung mittels einer Strahlung nahezu unabhängig von der Fertigungsgeschwindigkeit, so dass es sich um einen äußerst robusten Markierungsprozess handelt. Die Markierung ist in der Regel nur eine Frage der Bestrahlungsdosis.

Zudem entfallen durch das erfindungsgemäße Markierungsverfahren neben den Materialkosten für zusätzliche Markierungsstoffe auch die Kosten für deren Beschaffung, Lagerhaltung und Freigabe. Auch muss in der Regel nicht durch aufwendige Belegversuche ermittelt werden, ob eine Wechselwirkung der Markierungsstoffe mit unmarkierten Testelementen bestehen. Eine strategische Abhängigkeit von der Liefersicherheit bestimmter Markierungsstoffe entfällt ebenfalls.

Die genannten Vorteile machen sich insbesondere bemerkbar, wenn strahlungssensitive Eigenschaften eines Testmaterials des Testelements selbst ausgenutzt werden. Wie oben beschrieben, enthalten viele Arten von Testelementen derartige Testmaterialien, welche oft auch als "Nachweischemie" oder "Testchemie" bezeichnet werden und welche ausgewählt und eingerichtet sind, um bei Anwesenheit des mindestens einen Analyten in der Probe mindestens eine messbare Eigenschaft zu ändern, insbesondere eine messbare elektrische und/oder optische Eigenschaft. Diesbezüglich kann auf die aus dem Stand der Technik bekannten Testelemente und die dort verwendete Nachweischemie, welche beispielsweise auf Testfeldern der Testelemente aufgebracht ist, verwiesen werden.

Diese Nachweischemie bzw. dieses Testmaterial weist in der Regel selbst strahlungssensitive Eigenschaften auf. Dies ist insbesondere dann der Fall, wenn optische Testelemente verwendet werden, bei welchen der Nachweis des mindestens einen Analyten beispielsweise in Form einer Farbänderung oder einer Fluoreszenzänderung erfolgt. Auch bei anderen Arten von Testmaterialien lassen sich jedoch durch geeignete Auswahl der Strahlung nachweisbare, längerfristige Änderungen in den Testmaterialien herbeiführen.

Besonders bevorzugt ist es dabei, wenn die nachweisbaren Änderungen dergestalt sind, dass diese irreversibel sind. Zumindest sollte die nachweisbare Änderung jedoch eine Stabilität im Bereich von einigen Minuten, vorzugsweise einigen Stunden bis hin zu einigen Tagen, aufweisen. Verbleibt die Markierung auf den Testelementen, beispielsweise für eine spätere Auslesung durch ein entsprechend eingerichtetes Analysegerät, so ist sogar eine längere Stabilisierung erforderlich, beispielsweise eine optisch nachweisbare Änderung, welche über einen Zeitraum von mehreren Monaten bis hin zu Jahren stabil ist.

In dem Fall, in welchem das Testmaterial der Testelemente selbst als strahlungssensitives Material und somit zur Informationsspeicherung über mögliche Fehler eingesetzt wird, kann auf ein Aufbringen zusätzlicher Stoffe zur Markierung auf die Testelemente vollständig verzichtet werden. Damit machen sich die oben dargelegten Vorteile (Entfallen des Erfordernisses der Kompatibilitätsprüfung, Kostenverringerungen etc.) besonders positiv bemerkbar.

Wie oben beschrieben, kann zur Markierung der Testelemente, alternativ oder zusätzlich zur Nutzung der Testmaterialien bzw. der Testchemie, welche die analytische Funktionalität der Testfelder der Testelemente bedingt, auch eine Markierung abseits der Testfelder auf die Testelemente aufgebracht werden. Zu diesem Zweck kann beispielsweise ein Trägermaterial (beispielsweise ein Trägerband) der Testelemente selbst ein strahlungssensitives Material beinhalten, welches zur Markierung genutzt werden kann. Alternativ oder zusätzlich kann auch ein separates, von den Testmaterialien bzw. der Testchemie unabhängiges Markierungsmaterial mit strahlungssensitiven Eigenschaften auf die Testelemente aufgebracht werden. So können beispielsweise separate Markierungsfelder vorgesehen werden, welche ein strahlungssensitives Material beinhalten und welche somit markierbar sind. Auch beliebige Kombinationen sind denkbar, beispielsweise eine Markierung auf einem Testfeld (wobei das Testfeld dann ein erstes strahlungssensitives Material aufweist) und eine Markierung auf einem separaten Markierungsfeld (welches ein zweites strahlungssensitives Material aufweist). Entsprechend wird auch ein Testelement vorgeschlagen, welches zur Verwendung in einem derartigen Markierungsverfahren geeignet ist und welches mindestens ein derartiges, von dem bzw. den Testfeldern getrenntes Markierungsfeld mit einem strahlungssensitiven Material aufweist. Unter "getrennt" ist dabei eine funktionelle Trennung, jedoch nicht notwendigerweise eine strenge räumliche Trennung zu verstehen. So können beispielsweise auch Markierungsfelder an Testfelder angrenzen, teilweise mit diesen überlappen oder sogar in einem Schichtaufbau (beispielsweise unter den Testfeldern) gestapelt sein, so dass insgesamt die Funktionalität der Testfelder durch die Markierungsfelder nicht beeinflusst wird.

Die mindestens eine optisch nachweisbare Änderung in dem strahlungssensitiven Material, sei dieses nun in den Testfeldern selbst oder in separaten Markierungsfeldern aufgenommen, kann anschließend von einem geeigneten Sensor erfasst und als Ausschussmarkierung interpretiert werden. Auf mögliche Ausgestaltungen dieser Erfassung wird nachfolgend noch näher eingegangen.

Es sei darauf hingewiesen, dass nicht notwendigerweise eine Markierung der fehlerhaften Testelemente mit der Strahlung erfolgen muss, sondern dass auch ein "inverse" Markierung mittels der beschriebenen optisch nachweisbaren Änderung möglich ist. So kann beispielsweise jedes Testelement, welches als fehlerfrei erkannt wird, mittels der Strahlung markiert werden, wohingegen jedes als fehlerhaft erkannte Testelement unmarkiert verbleibt. Auch Zwischenstufen oder andere Arten der Fehlermarkierung sind möglich, beispielsweise indem durch die mindestens eine Strahlung Informationen über einen Qualitätsgrad des Testelements in das strahlungssensitive Material "eingeschrieben" werden, beispielsweise in Form eines Bit-Wertes. Der Informationsgehalt der Fehlermarkierung kann also unterschiedlich ausgestaltet sein und kann auf verschiedene Weise Informationen über eine Fehlerhaftigkeit (was auch eine Fehlerfreiheit einschließen kann) des jeweiligen markierten Testelementes (und/oder sogar anderer Testelemente, d.h. beispielsweise anderer Testelemente aus einer Charge) beinhalten. Wie oben beschrieben, kann es sich dabei beispielsweise um die Information "fehlerhaft", "nicht fehlerhaft", einen Qualitätsgrad oder eine ähnliche Information handeln. Diese Information über die Fehlerhaftigkeit des jeweiligen markierten Testelements wird in das strahlungssensitive Material mittels der mindestens einen Strahlung eingebracht.

Besonders bevorzugt ist es dabei, wenn die verwendete Strahlung, bei welcher es sich grundsätzlich auch um eine Partikelstrahlung, wie beispielsweise eine Neutronenstrahlung handeln könnte, eine elektromagnetische Strahlung umfasst. Insbesondere hat sich dabei die Verwendung einer ultravioletten Strahlung, also eine Strahlung im Wellenlängenbereich zwischen 1 nm und 400 nm als zweckmäßig erwiesen. Besonders bevorzugt ist der Wellenlängenbereich von 250 nm bis 400 nm, insbesondere der Wellenlängenbereich zwischen 350 nm und 380 nm, da dieser Wellenlängenbereich nicht nur gut mit der Sensitivität üblicher Testmaterialien übereinstimmt, sondern auch technisch gut realisierbar ist. Somit ist die bevorzugte Verwendung von UV-Licht, insbesondere in dem genannten bevorzugten Wellenlängenbereich, besonders in Zusammenwirkung mit der Verwendung der Nachweischemie als Informationsträger für die Fehlerinformation von Vorteil, da viele dieser Testmaterialien bzw. Nachweischemien, insbesondere für einen optischen Analyt-Nachweis, empfindlich auf UV-Strahlung reagieren. So können beispielsweise durch UV-Bestrahlung langfristig Mehrfachbindungen in organischen Bestandteilen der Testmaterialien zerstört werden, was wiederum beispielsweise durch einen Farbumschlag oder eine andere optisch nachweisbare Änderung zu erkennen sein kann.

Zur Erzeugung der mindestens einen Strahlung lassen sich vorzugsweise Glühlampen, Gasentladungslampen, Laser, Leuchtdioden, Blitzlampen oder eine Kombination derartiger Lichtquellen einsetzen, welche vorzugsweise wiederum zumindest teilweise im ultravioletten Spektralbereich emittieren.

Die optisch nachweisbare Änderung kann, entsprechend der obigen Beschreibung, beispielsweise eine Farbänderung, eine Änderung einer Lumineszenz (z.B. einer Fluoreszenz und/oder einer Phosphoreszenz), eine Änderung einer Reflektivität oder eine Kombination dieser Änderungen und/oder weiterer Änderungen umfassen.

Das vorgeschlagene Markierungsverfahren kann beispielsweise als "stand alone" Verfahren eingesetzt werden, um bereits fertig hergestellte Testelemente zu markieren. Besonders bevorzugt ist es jedoch, das vorgeschlagene Markierungsverfahren in einer der vorgeschlagenen Ausführungsformen im Rahmen eines Herstellungsverfahrens zur Herstellung von Testelementen der beschriebenen Art zu integrieren. Bei diesem Herstellungsverfahren wird eine Mehrzahl der Testelemente hergestellt und mindestens eines der Testelemente (vorzugsweise alle Testelemente und/oder Stichproben der hergestellten Testelemente) werden einer Fehlerprüfung unterzogen.

Beispielsweise kann diese Fehlerprüfung, wie oben dargelegt, eine optische Prüfung (z.B. eine Farb- und/oder Fluoreszenzmessung), eine Sichtprüfung (z.B. im Rahmen einer Mustererkennung, insbesondere mittels eines digitalen Bildverarbeitungsprogramms), eine Fluoreszenzprüfung oder ähnliche Arten von Fehlerprüfungen oder Kombinationen von Fehlerprüfungen beinhalten. Auf diese Weise kann zum Beispiel erkannt werden, ob die Testelemente selbst (z.B. ein Trägerband der Testelemente) verformt sind, ob eine Nachweischemie korrekt aufgebracht wurde (z.B. ob ein Testfeld aufgebracht wurde und/oder ob dieses Testfeld beim Aufbringen deformiert wurde) oder Ähnliches. Ein weiteres Beispiel eines Testkriteriums der Fehlerprüfung stellt die Homogenität der Testfelder dar. So kann beispielsweise überprüft werden, ob eine Testchemie homogen und/oder mit konstanter Schichtdicke auf einem Testfeld enthalten ist. Diese Homogenität kann beispielsweise mittels einer optischen Kontrolle im sichtbaren, infraroten oder ultravioletten Spektralbereich überprüft werden. Alternativ oder zusätzlich kann auch eine Schichtdicken- und/oder Homogenitätskontrolle beispielsweise mittels ellipsometrischer Verfahren, Transmissionsverfahren, mechanischer Abtastverfahren oder anderer gängiger Schichtdickenmessverfahren, welche vorzugsweise eine Fläche abtasten oder gleichzeitig erfassen können, durchgeführt werden. So können beispielsweise Fehler bei der Herstellung der Testfelder (beispielsweise Fehler bei einem Druckverfahren) festgestellt werden. Hierbei können Toleranzschwellen für die Homogenität (beispielsweise "erlaubte" Bereiche für die Schichtdickenwerte innerhalb eines Testfeldes) vorgegeben werden, um die Testfelder bzw. Testelemente als fehlerfrei oder fehlerhaft zu qualifizieren. Ein weiteres Beispiel für ein Testkriterium der Fehlerkontrolle ist eine Abstandskontrolle, bei welcher (beispielsweise auf optischem Wege, beispielsweise mittels einer Mustererkennung) Abstände bestimmter Elemente auf und/oder in den Testelementen mit Sollwerten verglichen werden. So kann beispielsweise überprüft werden, ob Testfelder einen vorgegebenen Anstand untereinander und/oder einen vorgegebenen Abstand zu bestimmten Markierungen aufweisen. Auch zwei- oder dreidimensionale Abstandsüberwachungen sind möglich. Weiterhin können auch Toleranzbereiche vorgegeben werden, innerhalb derer die gemessenen Abstände noch tolerierbar sind und außerhalb derer die Testelemente als fehlerhaft erkannt werden. Weiterhin können, alternativ oder zusätzlich, auch beispielsweise elektrische oder elektrochemische Messverfahren eingesetzt werden, wie beispielsweise Widerstandsmessungen, Impedanzmessungen oder ähnliche Arten von Messungen. Auch eine Kombination der genannten Fehlerprüfungsarten und/oder weitere Arten der Fehlerprüfung sind einsetzbar und sind dem Fachmann bekannt.

Die Fehlerprüfung kann bereits während der Herstellung der Testelemente erfolgen und kann somit beispielsweise in eine Fertigungsstraße integriert werden. Alternativ oder zusätzlich kann die Fehlerprüfung auch zeitlich der Herstellung nachgeordnet sein.

Für die Herstellung der Testelemente können herkömmliche, dem Fachmann bekannte Verfahren eingesetzt werden, beispielsweise Dickschichtverfahren, halbleitertechnische Verfahren, Druckverfahren oder eine Kombination dieser/oder weiterer üblicher Verfahrensschritte. Die Herstellung der Testelemente ist dem Fachmann bekannt. Insbesondere kann ein Bandverfahren verwendet werden, wie unten näher ausgeführt wird. Weiterhin kann vorzugsweise das Testmaterial separat hergestellt werden und anschließend (beispielsweise in einem Laminierprozess) auf das Band bzw. eine andere Art von Träger aufgebracht werden.

Bei oder nach der Fehlerprüfung wird entschieden, ob das Testelement fehlerhaft ist. Analog zur obigen Beschreibung des Begriffs der "Fehlerinformation" bzw. "Fehlermarkierung" ist der Begriff "fehlerhaft" weit auszulegen. So kann dieser beispielsweise eine "digitale" Fehlerhaftigkeit, beispielsweise in Form einer Fehlerhaftigkeit bzw. einer Fehlerfreiheit (wobei jeweils auch Toleranzschwellen vorgegeben sein können, beispielsweise Schwellen für tolerierbare Fehler), oder es kann auch eine Anzahl von Zwischeninformationen beinhaltet sein. Diese Zwischeninformationen können beispielsweise wiederum Qualitätsstufen des jeweils geprüften Testelements umfassen, welche z.B. wiederum in einem Bit-Wert ausgedrückt werden können.

Anschließend an die Fehlerprüfung oder bereits bei der Fehlerprüfung kann wiederum das Testelement mit einer Fehlermarkierung versehen werden, welche eine Information über eine Fehlerhaftigkeit des jeweiligen Testelements beinhaltet. Bezüglich dieser Information über die Fehlerhaftigkeit kann auf die obige Beschreibung verwiesen werden. Bei dieser Markierung des mindestens einen der Testelemente wird ein Markierungsverfahren gemäß der obigen Beschreibung in einer der dargestellten Ausführungsformen verwendet.

Beispielsweise kann wiederum, wie oben beschrieben, mindestens ein Testmaterial (Testchemie) des Testelements als Informationsträger für die Markierung dienen. Beispielsweise kann, wie ebenfalls oben dargelegt, das Markierungsverfahren derart ausgestaltet sein, dass diese Nachweischemie oder Testchemie entsprechend verfärbt wird. Auf diese Weise kann insbesondere ein Testelement oder ein Teil des Testelements (z.B. ein bestimmtes Testfeld) unbrauchbar gemacht werden, so dass ein falscher Messwert beim Analytnachweis ausgeschlossen werden kann.

Diese mindestens eine Information, welche in der Fehlermarkierung enthalten ist, kann anschließend weiter verwendet werden. So können insbesondere in einem Aussortierungsschritt markierte Testelemente aussortiert werden. Dies ist naturgemäß insbesondere bei Herstellungsverfahren von Vorteil, bei welchen Testelemente während oder nach der Herstellung vereinzelt werden, so dass die als fehlerhaft markierten Testelemente (d.h. je nach Art der aufgebrachten Fehlerinformation markierte oder unmarkierte Testelemente oder Testelemente, deren Fehlerinformation einen unzureichenden Gütegrad indiziert) aussortiert und beispielsweise entsorgt werden können. Beispielsweise können mehrere Testelemente als Bandware auf einem gemeinsamen Trägerband hergestellt werden, wobei die Bandware anschließend beispielsweise in einer Bandkassette aufgenommen werden kann. Als fehlerhaft erkannte Testelemente können beispielsweise vor Aufnahme in der Bandkassette ausgeschnitten werden, und das übrige, fehlerfreie Band kann anschließend in einem Splicing-Schritt wieder zusammengefügt werden. Sol lassen sich auch bei Bandware fehlerhafte Testelemente vermeiden. Alternativ oder zusätzlich kann die Bandware anschließend auch vereinzelt werden, wobei fehlerhafte Testelemente aussortiert werden können. Auf diese Weise kann jeweils im Wesentlichen sichergestellt werden, dass alle in den Handel gelangenden Testelemente fehlerfrei sind.

Die Bandware kann auch derart ausgestaltet sein, dass diese (wie eingangs bei der Beschreibung des Standes der Technik dargelegt) in einer Bandkassette aufgenommen wird. Hierbei kann ein Testband eine Vielzahl von Testelementen beinhalten, welche beispielsweise nacheinander verwendet werden können. Dabei können beispielsweise fehlerhafte Testelemente besonders markiert werden, so dass diese entweder nicht verwendet werden können, von einem Analysegerät als fehlerhaft erkannt werden oder auf andere Weise in ihrer Benutzung eingeschränkt sind. Alternativ oder zusätzlich ist es jedoch bevorzugt, beispielsweise durch den oben beschrieben Schneideprozess mit anschließendem Aussortieren und Splicing des verbleibenden Bandes, das Band so zusammenzustellen, dass dieses keine fehlerhaften Testelemente umfasst.

Das Herstellungsverfahren kann weiterhin vorzugsweise derart ausgestaltet sein, dass dieses optional nach dem mindestens einen Verfahrensschritt, in welchem die mindestens eine Fehlermarkierung aufgebracht wird, einen Kontrollschritt umfasst. Bei diesem Kontrollschritt kann überprüft werden, ob die Fehlermarkierung korrekt aufgebracht wurde. Beispielsweise kann dies dadurch erfolgen, dass die mindestens eine optisch nachweisbare Änderung in dem strahlungssensitiven Material überwacht wird und mit einem Sollwert verglichen wird. Beispielsweise kann dieser Sollwert in einem Datenspeicher abgespeichert sein, beispielsweise einem Datenspeicher (z.B. einem Schieberegister), welcher auch für die Fehlermarkierung selbst verwendet wird. Auf diese Weise kann unmittelbar überprüft werden, ob die Fehlermarkierung korrekt durchgeführt wurde. Wird erkannt, dass dies nicht der Fall ist, so kann beispielsweise eine Warnung an ein Bedienpersonal ausgegeben werden und/oder das fehlerhaft markierte Testelement kann erneut markiert und/oder ausgesondert werden. Verschiedene weitere Möglichkeiten sind denkbar.

Entsprechend dem oben dargelegten Markierungsverfahren und dem Herstellungsverfahren in jeweils einer der beschriebenen Ausführungsformen wird weiterhin erfindungsgemäß eine Markierungsvorrichtung zur Markierung fehlerhafter Testelemente vorgeschlagen. Die Markierungsvorrichtung kann insbesondere eingerichtet sein, um ein Markierungsverfahren der beschriebenen Art in einer der Ausführungsvarianten durchzuführen. Entsprechend weist die Markierungsvorrichtung mindestens eine Strahlungsquelle auf, um die Testelemente mit mindestens einer Strahlung zu beaufschlagen. Für die Wirkung dieser Strahlung sowie die möglichen Arten der Markierung in der Markierungsvorrichtung kann auf die obige Beschreibung verwiesen werden.

Weiterhin kann die Markierungsvorrichtung mindestens eine Testvorrichtung umfassen, welche eingerichtet ist, um mindestens eines der Testelemente einer Fehlerprüfung zu unterziehen. Vorzugsweise werden alle Testelemente oder Stichproben der Testelemente dieser Fehlerprüfung unterzogen. Bezüglich der möglichen Ausgestaltung der Fehlerprüfung kann ebenfalls auf die obige Beschreibung verwiesen werden. Die Markierungsvorrichtung ist eingerichtet, um zu erkennen und/oder zu entscheiden, ob das Testelement fehlerhaft ist. Bezüglich des Begriffes "fehlerhaft" und seiner möglichen Bedeutungen kann ebenfalls auf die obige Definition verwiesen werden.

Entsprechend der obigen Beschreibung der möglichen Markierungsverfahren kann die Strahlungsquelle beispielsweise wiederum Glühlampen, Gasentladungslampen, Laser, Leuchtdioden, Blitzlampen oder Kombinationen dieser und/oder anderer Strahlungsquellen, insbesondere zur Erzeugung von ultravioletter Strahlung gemäß der obigen Definition und in dem oben beschriebenen bevorzugten Wellenlängenbereich, aufweisen. Besonders bevorzugt ist es dabei, um eine hohe Parallelität und somit einen hohen Durchsatz des Markierungsverfahrens zu bewirken, wenn die Markierungsvorrichtung eine Vielzahl von modular aufgebauten Einzellichtquellen aufweist. Insbesondere kann es sich hierbei um eine Vielzahl von modular aufgebauten Lichterzeugereinheiten zur Erzeugung von ultraviolettem Licht handeln. Diese modular aufgebauten Lichterzeugereinheiten können beispielsweise eine Vielzahl von identischen Lichterzeugereinheiten umfassen, welche beispielsweise in einem Schaltschrank der Markierungsvorrichtung angeordnet sein können. Beispielsweise können diese Lichterzeugereinheiten in Form von identischen Einschüben in dem Schaltschrank aufgenommen sein.

Die Markierungsvorrichtung kann mindestens eine Applikationsposition umfassen, in welcher die Testelemente ganz oder teilweise mit der Fehlermarkierung versehen werden. Um einen hohen Parallelisierungsgrad und somit einen hohen Durchsatz zu erzielen, können auch mehrere Applikationspositionen vorgesehen sein. So kann beispielsweise ein Testmaterial (Testchemie, Nachweischemie) in Form eines oder mehrerer Testfelder auf jeweils ein Testelement aufgebracht sein, wobei für jedes Testfeld eine eigene Applikationsposition vorgesehen sein kann.

Besonders bevorzugt ist es dabei, wenn die Markierungsvorrichtung weiterhin mindestens einen Wellenleiter aufweist, welcher eingerichtet ist, um die Strahlung von der mindestens einen Strahlungsquelle zu der Applikationsposition zu leiten. Bei diesem mindestens einen Wellenleiter kann es sich, je nach Art der eingesetzten Strahlung, insbesondere um einen Lichtwellenleiter handeln. Wenn, wie besonders bevorzugt, ultraviolettes Licht als Strahlung eingesetzt wird, so sollte dieser Lichtwellenleiter auf die jeweils verwendete Wellenlänge angepasst sein. Dabei können starre oder flexible Wellenleiter verwendet werden, beispielsweise Wellenleiter mit einem Kunststoff- und/oder Glasmaterial. Derartige Wellenleiter können beispielsweise als starre Kunststoff-Wellenleiter realisiert sein. Besonders bevorzugt ist es jedoch, wenn der Wellenleiter mindestens einen Faserlichtleiter umfasst, also einen flexiblen Lichtleiter. Dabei können Glasfasern und/oder, was besonders bevorzugt ist, Kunststofffaserlichtleiter eingesetzt werden. Jeweils sind, je nach gewünschten Eigenschaften der Strahlung, Multimode- oder Singlemode-Fasern einsetzbar.

Beispielsweise können mehrere Faserlichtleiter, zum Beispiel Kunststofffaserlichtleiter, zu Faserbündeln zusammengefasst sein. So können beispielsweise die oben beschriebenen bevorzugten UV-Lichterzeugereinheiten, welche sich vorzugsweise in einem Schaltschrank der Markierungsvorrichtung befinden, über ein oder mehrere Faserbündel mit der mindestens einen Applikationsposition verbunden sein.

Werden Faserbündel verwendet, so ist es insbesondere bevorzugt, wenn die Markierungsvorrichtung mindestens einen Querschnittswandler aufweist. Dieser Querschnittswandler ist eingerichtet, um eine Vielzahl von Faserlichtleitern mindestens eines der Faserbündel derart zu halten, dass die Faserenden der Faserlichtleiter in der Applikationsposition in einem vorgegebenen Muster angeordnet sind. Auf diese Weise können beispielsweise durch entsprechende Anordnung der Faserenden zu einem Muster der Durchsatz der Testelemente erhöht werden, da parallel mehrere Testelemente beaufschlagt werden können, es können Intensitäten und Strahlungsdosen (durch gleichzeitige Beaufschlagung aus mehreren Fasern und/oder durch Beaufschlagung aus mehreren Fasern nacheinander) erhöht werden, und es könnten auch auf einem einzelnen Testelement mehrere Fehlermarkierungen gleichzeitig eingeschrieben werden.

Diese Ausgestaltung der Markierungsvorrichtung macht sich insbesondere dann vorteilhaft bemerkbar, wenn, wie es ebenfalls bevorzugt ist, die Testelemente in einem kontinuierlichen Prozess hergestellt werden. Beispielsweise können die Testelemente in Form eines kontinuierlichen Testelementbandes hergestellt werden, welches nachher, wie oben beschrieben, vereinzelt wird oder welches als Ganzes oder abschnittsweise in einer Bandkassette aufgenommen werden kann. Dabei kann das Muster der Faserenden, welches durch den Querschnittswandler erzeugt wird, eine gleichzeitige Bestrahlung in der Applikationsposition sowohl beispielsweise parallel zu einer Laufrichtung des Bandes als auch, alternativ oder zusätzlich, senkrecht zur Laufrichtung des Bandes möglich machen. Auch können mehrere Bänder gleichzeitig bearbeitet werden, beispielsweise in dem mehrere Bänder mit Testelementen parallel erzeugt werden. Beispielsweise kann bei der Herstellung von Bandware zunächst ein breites Band mit mehreren jeweils parallel angeordneten Testelementen hergestellt werden, welches dann (beispielsweise in einem Schneideprozess) in mehrere Teilbänder zerschnitten wird.

Die Markierungsvorrichtung kann in der Applikationsposition einen Führungstisch aufweisen, in dem zwei, vorzugsweise mindestens fünf, Testelemente gleichzeitig mit der Strahlung beaufschlagbar sind, d.h. markiert werden können. Beispielsweise können fünf Bänder oder Teilbänder mit Testelementen gleichzeitig durch den Führungstisch gefahren werden.

Für das vorgegebene Muster, welches durch den Querschnittswandler hergestellt wird, werden insbesondere ein Linienmuster (z.B. mit einer Linie parallel und/oder senkrecht zur Bewegungsrichtung des Bandes), ein Matrixmuster oder ein Rechteckmuster bevorzugt. Auch andere Muster sind selbstverständlich möglich. Auch ist es möglich, mehrere Querschnittswandler parallel zueinander anzuordnen, beispielsweise mehrere Querschnittswandler mit jeweils linienförmig angeordneten Faserenden. Dies ist insbesondere in der oben beschriebenen Ausführungsvariante, bei welcher mehrere Testbänder mit Testelementen parallel erzeugt werden von Vorteil, da auf diese Weise beispielsweise jedem Band oder Teilband ein Querschnittswandler mit einem Faserenden-Muster zugeordnet sein kann.

Alternativ oder zusätzlich kann die Markierungsvorrichtung weiterhin mindestens eine Strahlformungsoptik zur Strahlformung der Strahlung aufweisen. Beispielsweise können Linsensysteme vorgesehen sein, um die Markierungen bzw. die Form der Markierungen auf den Testelementen in eine gewünschte Form zu bringen, beispielsweise eine Linien-oder Rechteckform.

Neben der Markierungsvorrichtung wird weiterhin eine Herstellungsvorrichtung zur Herstellung von Testelementen vorgeschlagen, welche insbesondere eingerichtet sein kann, um Testelemente nach dem oben beschriebenen Herstellungsverfahren in einer der beschriebenen Verfahrensvarianten herzustellen. Die Herstellungsvorrichtung weist eine Produktionsvorrichtung zur Herstellung einer Mehrzahl von Testelementen auf. Beispielsweise können für diese Produktionsvorrichtung, wie oben beschrieben, aus dem Stand der Technik bekannte Herstellungsvorrichtungen eingesetzt werden, beispielsweise Herstellungsvorrichtungen, welche mit Halbleiter- und/oder Dickschichtprozessen und/oder Druckverfahren und/oder Klebeverfahren arbeiten. Insbesondere kann die Produktionsvorrichtung eingerichtet sein, um kontinuierlich eine Vielzahl von Testelementen herzustellen, beispielsweise wiederum in Form eines Bandprozesses, wobei auf einem gemeinsamen Band eine Vielzahl von Testelementen aufgenommen ist. Diese Testelemente können anschließend vereinzelt werden, oder das Band kann als solches verwendet werden, beispielsweise in einer Bandkassette.

Weiterhin weist die Herstellungsvorrichtung mindestens eine Markierungsvorrichtung gemäß einer der oben beschriebenen Ausführungsformen auf. Daneben kann die Herstellungsvorrichtung weiterhin mindestens eine Sortiervorrichtung zum Aussortieren von als fehlerhaft markierten Testelementen aufweisen. Wird eine Bandware hergestellt, beispielsweise für eine Bandkassette mit Testelementen, so ist es bevorzugt, wenn die Herstellungsvorrichtung weiterhin eine Schneide- und Splicingvorrichtung aufweist, also eine Vorrichtung, welche fehlerhafte Testelemente aus einem Band ausschneiden kann, um dieses Band anschließend wieder zusammenzukleben oder auf andere Weise zusammenzufügen, beispielsweise durch Laminieren. Auf diese Weise kann ein lückenloses Band mit fehlerfreien Testelementen hergestellt werden. Wiederum sind die Begriffe "fehlerhaft" und "fehlerfrei" dabei weit zu fassen, wobei auf die obige Beschreibung verwiesen werden kann. Beispielsweise können wiederum Toleranzschwellen vorgegeben werden. Je nachdem ob die Testelemente bei oder nach der Herstellung vereinzelt werden, können fehlerhafte Testelemente auch, anstatt aussortiert zu werden, beispielsweise auf einem Band verbleiben und lediglich später beispielsweise durch Auslesen der Markierung unbenutzt bleiben (siehe auch unten).

Die Herstellungsvorrichtung kann insbesondere weiterhin mindestens eine Testvorrichtung aufweisen. Diese Testvorrichtung ist eingerichtet, um die Testelemente einer Fehlerprüfung zu unterziehen. Dabei können die oben bereits beschriebenen Fehlerprüfungsverfahren eingesetzt werden, also beispielsweise elektrische, elektrochemische oder optische Messverfahren oder eine Kombination dieser Fehlerprüfungsverfahren. So kann beispielsweise die Testvorrichtung eine Kamera umfassen, mittels derer die Testelemente aufgenommen werden. Alternativ oder zusätzlich kann beispielsweise auch ein Bilderkennungssystem vorgesehen sein, welches beispielsweise eingerichtet ist, um Abweichungen eines Testfeldes und/oder einer Form der Testelemente von einer vorgegebenen Norm (z.B. um mehr als eine vorgegebene Toleranzschwelle) zu detektieren. Alternativ oder zusätzlich können auch eine oder mehrere Testlichtquellen eingesetzt werden, beispielsweise um Fluoreszenzeigenschaften und/oder Farbeigenschaften und/oder Reflexionseigenschaften des Testelements zu überprüfen, beispielsweise Eigenschaften einer Testchemie und/oder eines oder mehrerer Testfelder. Weiterhin können, alternativ oder zusätzlich, beispielsweise auch, wie oben ebenfalls dargelegt, eine oder mehrere Widerstandsmessvorrichtungen vorgesehen sein und/oder eine oder mehrere Impedanzmessvorrichtungen und/oder ähnliche Vorrichtungen zur elektrischen oder elektrochemischen Fehlerüberprüfung. Weiterhin kann die Herstellungsvorrichtung, insbesondere die Testvorrichtung, alternativ oder zusätzlich einen oder mehrere Datenspeicher umfassen, wobei es sich um flüchtige und/oder nicht flüchtige Datenspeicher handeln kann. Besonders bevorzugt ist es dabei im Rahmen einer Serienfertigung, wenn dieser mindestens eine Datenspeicher mindestens einen Schieberegister umfasst. Beispielsweise kann wiederum ein kontinuierlicher oder schrittweiser Herstellungsprozess verwendet werden, bei welchem an einer Stelle mittels der Fehler-überprüfungsvorrichtung die Fehlerüberprüfung stattfindet, wobei die Ergebnisse dieser Fehlerüberprüfung in den Datenspeicher, insbesondere das Schieberegister, eingetragen werden können. Auf diese Weise können nacheinander eine Vielzahl von Testelementen getestet werden, so dass anschließend, durch entsprechendes Auslesen des Schieberegisters, beispielsweise von der Markierungsvorrichtung ein sich gerade in der Markierungsvorrichtung befindliches Testelement (z.B. ein oder mehrere Testelemente in einer Applikationsposition) jeweils der Information bzw. den Informationen im Schieberegister zugeordnet werden können. Auf diese Weise kann dann mittels der Markierungsvorrichtung eine Markierung der Testelemente erfolgen. Analog kann die Information später auch für ein Aussortieren der Testelemente genutzt werden.

Weiterhin kann die Herstellungsvorrichtung, analog zur obigen Beschreibung eines bevorzugten Verfahrens, bei welchem ein Kontrollschritt durchgeführt wird, mindestens eine Kontrollvorrichtung umfassen, welche eingerichtet ist, um zu überprüfen, ob in der Markierungsvorrichtung korrekt markiert wurde. So kann beispielsweise die Kontrollvorrichtung wiederum mit Daten eines Datenspeichers beaufschlagt werden, beispielsweise eines Schieberegisters, wobei vorzugsweise dieselben Daten verwendet werden, auf welche auch die Markierungsvorrichtung zugreifen kann und aufgrund derer die Fehlermarkierungen der Testelemente aufgebracht werden. Auf diese Weise lässt sich durch eine Gegenkontrolle zumindest weitgehend sicherstellen, dass die Markierungsvorrichtung korrekt arbeitet, und es können beispielsweise Ausfälle in einzelnen Lichtquellen oder andere Arten von Fehlfunktionen erkannt werden. Wird eine derartige Fehlfunktion erkannt, so kann beispielsweise die Herstellungsvorrichtung derart ausgestaltet sein, dass diese die fehlerhaft markierten Testelemente aussondert und/oder eine Warnung an Bedienpersonal ausgibt oder ähnliche Aktionen durchführt. Die Kontrollvorrichtung kann beispielsweise eine oder mehrere Photodioden und/oder andere Arten von Photodetektoren umfassen, um die mindestens eine optisch nachweisbare Änderung in dem strahlungssensitiven Material zu überwachen. Auch eine oder mehrere Lichtquellen können vorgesehen sein, beispielsweise um eine Funktion der Photodetektoren durch entsprechende Beaufschlagung des strahlungssensitiven Materials zu unterstützen.

Weiterhin wird ein analytisches Testgerät zum Nachweis mindestens eines Analyten in einer Probe vorgeschlagen, welches mindestens ein Testelement umfasst, dass nach dem oben beschriebenen Herstellungsverfahren in einer der dargestellten Verfahrensvarianten hergestellt ist. Als Gegenstück zu der gegebenenfalls auf dem Testelement vorhandenen Fehlermarkierung weist das analytische Testgerät eine Abfragevorrichtung auf, welche ausgestaltet ist, um zu erkennen, ob das Testelement markiert ist. Zu diesem Zweck ist die Abfragevorrichtung eingerichtet, um die mindestens eine optisch nachweisbare Änderung in dem strahlungssensitiven Material des Testelements zu erkennen.

Beispielsweise kann dieses analytische Testgerät weiterhin eine Ansteuer- und Auswertevorrichtung umfassen, um mittels des mindestens einen Testelements eine quantitativen und/oder qualitativen Nachweise des mindestens einen Analyten in der Probe zu führen. Besonders bevorzugt ist es, wenn das analytische Testgerät eingerichtet ist, um eine Blutzuckerkonzentration zu bestimmen. Dabei können einzelne Testelemente verwendet werden, mehrere Testelemente, welche in einem Magazin aufgenommen sind (wobei z.B. jeweils ein Testelement dem Magazin entnommen wird), oder Testelemente, welche in einer Bandkassette aufgenommen sind. Auch andere Arten von Testelementen sind möglich, beispielsweise implantierbare Testelemente.

Die Abfragevorrichtung ist somit ausgestaltet, um die Fehlermarkierung des mindestens einen Testelementes, vorzugsweise des mindestens einen Testelements, welches gerade verwendet wird oder verwendet werden soll, abzufragen. Wird dabei, entsprechend der in der Fehlermarkierung enthaltenen Information, erkannt, dass das Testelement fehlerhaft ist, so können verschiedene Aktionen durchgeführt werden. Unter anderem kann eine Warnung an einen Benutzer des analytischen Testgerätes ausgegeben werden, beispielsweise über ein Display, optische Anzeigenelemente (z.B. Warnleuchten), über akustische Signale oder über eine Kombination dieser Möglichkeiten. Alternativ oder zusätzlich kann auch ein Test mit dem markierten Testelement verhindert werden. Wiederum alternativ oder zusätzlich kann bewirkt werden, dass ein neues Testelement aus einer Mehrzahl von in einem Magazin aufgenommenen Testelementen angefordert wird. Beispielsweise kann es sich, wie oben dargelegt, bei diesen Testelementen in diesem Magazin um einzelne Testelemente handeln oder um flexibel oder starr verbundene Testelemente. Beispielsweise kann auch eine Bandkassette verwendet werden, so dass beispielsweise bei einer Fehlererkennung von einem Testfeld auf ein nächstes Testfeld weiter gespult werden kann.

### Ausführungsbeispiele

Weitere Einzelheiten und Merkmale der Erfindung ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: einen schematischen Aufbau eines Ausführungsbeispiels einer erfindungsgemäßen Herstellungsvorrichtung mit einer Markierungsvorrichtung;
- Figur 2: ein Ausführungsbeispiel eines Querschnittswandlers;
- Figur 3: ein bandförmiges Testelement mit mehreren Testfeldern, aus denen ein Testfeld markiert ist;
- Figur 4: ein Ausführungsbeispiel eines analytischen Testgerätes;
- Figur 5: ein Ausführungsbeispiel eines Verfahrens zur Bestimmung einer Analytkonzentration mittels eines analytischen Testgeräts; und
- Figur 6: ein Ausführungsbeispiel einer Bandkassette zur Verwendung in dem analytischen Testgerät gemäß Figur 4.

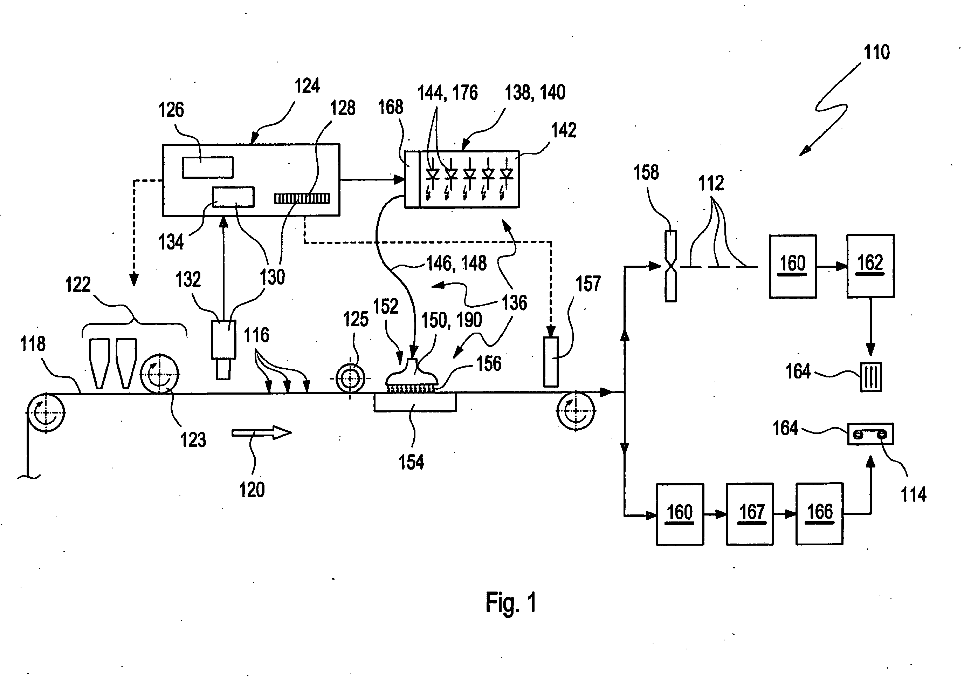

In Figur 1 ist ein Ausführungsbeispiel einer erfindungsgemäßen Herstellungsvorrichtung 110 zur Herstellung von Testelementen zum Nachweis mindestens eines Analyten in einer Probe dargestellt. Die Testelemente sind in Figur 1 mit den Bezugsziffern 112, 114 und 116 bezeichnet, je nach dem Produktionszustand bzw. Stadium, in welchem sich diese Testelemente innerhalb der Herstellungsvorrichtung 110 befinden bzw. je nach Ausgestaltung dieser Testelemente.

Die einzelnen Elemente der Herstellungsvorrichtung 110 sind in Figur 1 lediglich symbolisch angeordnet. Dabei ist ein kontinuierlicher Herstellungsprozess dargestellt, bei welchem die Testelemente zunächst als Bandware 116 auf einem Endlosband 118 hergestellt werden. Beispielsweise kann es sich bei diesem Endlosband 118 um ein Papierband handeln, insbesondere ein mehrlagiges, beschichtetes Papierband. Alternativ oder zusätzlich sind auch andere Trägermaterialien einsetzbar, beispielsweise Kunststoffbänder, Keramikbänder, Verbundmaterialien und ähnliche Trägermaterialien. Beispielsweise lässt sich eine Polyethylenfolie, eine PET-Folie oder ein anderes Folienmaterial als Trägermaterial einsetzen. Die Produktionsrichtung vom Ausgangsmaterial zu den fertigen Testelementen 112,114 erfolgt dabei in Figur 1 von links nach rechts, was in Figur 1 symbolisch mit dem Pfeil 120 bezeichnet ist. Alternativ zu kontinuierlichen Herstellungsprozessen, wie in Figur 1 dargestellt, sind jedoch auch diskontinuierliche Prozesse möglich, beispielsweise Batch-Prozesse, bei welchen Testelemente einzeln oder chargenweise hergestellt werden. Dies kann auch bei bandförmiger Ware erfolgen, beispielsweise indem Bandware abschnittsweise auf Trägerrollen aufgewickelt wird, um zwischengelagert zu werden und/oder um an andere Verfahrensabschnitte übergeben zu werden. Auch kann der Herstellungsprozess unterbrochen sein, so dass beispielsweise die Testelemente in verschiedenen Fertigungsstufen an unterschiedlichen Stellen (zum Beispiel an unterschiedlichen Standorten) gefertigt werden. Außerdem ist die dargestellte Reihenfolge der einzelnen Prozessschritte des Herstellungsprozesses der Herstellungsvorrichtung 110 nicht zwingend erforderlich, so dass beispielsweise auch ein Testen und Markieren von halbfertigen Testelementen erfolgen kann, so dass sich beispielsweise an ein Testen und Markieren weitere Produktionsschritte anschließen können.

Die Herstellungsvorrichtung 110 weist eine Produktionsvorrichtung 122 auf, welche in Figur 1 lediglich symbolisch angedeutet ist. Hier kann beispielsweise das Endlosband 118 8 mit Testfeldern versehen werden, in welchen jeweils ein Testmaterial (im Folgenden auch als Testchemie oder Nachweischemie bezeichnet) auf das Endlosband 118 aufgebracht werden kann. Dieses Aufbringen kann beispielsweise durch Aufkleben erfolgen, was in Figur 1 symbolisch durch eine Klebestation 123 angedeutet ist. Hier können, beispielsweise durch einen Klebe- oder Laminierprozess, jeweils Testfelder mit dem Testmaterial (Testchemie, Nachweischemie) auf das Trägermaterial aufgeklebt werden. Neben dem Testmaterial können weiterhin auch Markierungen auf dem Trägerband 118 aufgebracht, beispielsweise aufgedruckt werden, beispielsweise Markierungen, die der späteren Positionierung der Testelemente 116 in einem analytischen Testgerät dienen.

Die Produktionsvorrichtung 122 kann verschiedene einzelne Vorrichtungen zur Herstellung der Testelemente 116 umfassen, beispielsweise Beschichtungsdüsen, Druckeinrichtungen (z.B. Sieb-, Schablonen-, Tampon-, Inkjet- oder Flexodruckeinrichtungen) oder auch andere Arten von Produktionseinrichtungen oder Kombinationen von Produktionseinrichtungen. Auch können beispielsweise mehrere Bänder als Trägermaterialien zusammengeführt werden. Auch ist es möglich, die Herstellung teilweise in getrennte Verfahren zu unterteilen, so dass beispielsweise Halbfertigprodukte, wie ein bedrucktes Trägerband, der Produktionsvorrichtung 122 zugeführt werden. Auf die Einrichtungen der Produktionsvorrichtung 122, welche dem Fachmann aus der Herstellung von Testelementen bekannt sind, soll hier nicht näher eingegangen werden.

Besonders bevorzugt ist es dabei, wenn die Produktionsvorrichtung 122 derart eingerichtet ist, dass diese ein breites Trägerband 118 erzeugt, auf welchem mehrere Testelemente 116 parallel zueinander angeordnet sind. Beispielsweise kann das Band 118 zunächst so ausgestaltet sein, dass fünf derartiger Testelementbahnen nebeneinander angeordnet sind. Diese können dann beispielsweise durch Längsschnitte in einer Schneidevorrichtung (in Figur 1 symbolisch mit 125 bezeichnet) in einzelne Testelementbänder geschnitten werden. Dieses Zerschneiden kann in verschiedenen Stadien des in Figur 1 gezeigten Herstellungsprozesses erfolgen. Auch eine andere Ausgestaltung ist jedoch möglich, also beispielsweise eine Ausgestaltung mit lediglich einem Trägerband 118.

Die Herstellungsvorrichtung 110 weist weiterhin in diesem Ausführungsbeispiel eine zentrale Steuereinheit 124 auf. Sinngemäß kann diese Steuereinheit 124 alternativ auch dezentral aufgebaut sein, so dass die Herstellungsvorrichtung 110 beispielsweise mehrere Steuerungen umfassen kann. Diese Steuerungen können miteinander verbunden sein oder können auch autark arbeiten. Die zentrale Steuereinheit 124 umfasst in diesem Ausführungsbeispiel mindestens einen Prozessor 126 und mindestens einen Datenspeicher 128. Dieser mindestens eine Datenspeicher 128 kann insbesondere ein Schieberegister umfassen. Die zentrale Steuereinheit 124 kann insbesondere programmtechnisch ausgestaltet sein, um ein Herstellungsverfahren, beispielsweise ein Herstellungsverfahren gemäß einem der oben beschriebenen Ausführungsbeispiele, zu steuern.

Weiterhin umfasst die Herstellungsvorrichtung 110 eine oder mehrere Testvorrichtungen 130. Auch diese Testvorrichtung 130 ist in Figur 1 lediglich symbolisch dargestellt. So kann diese Testvorrichtung 130 beispielsweise eine oder mehrere Kameras 132 umfassen. Diese Kameras können beispielsweise ausgestaltet sein, um die Testfelder auf den in diesem Verfahrensstadium beispielsweise noch als Bandware ausgestalteten Testelementen 116 zu beobachten. Die Testvorrichtung 130 kann weiterhin ein Bilderkennungssystem 134 umfassen, welches beispielsweise dezentral oder (wie in Figur 1 symbolisch dargestellt) als Bestandteil der zentralen Steuereinheit 124 ausgebildet sein kann. Das Bilderkennungssystem 134 kann insbesondere ein oder mehrere Bilderkennungs-Softwaremodule umfassen, welche beispielsweise auf dem mindestens einen Prozessor 126 ablaufen können. Das Bilderkennungssystem 134 kann eingerichtet sein, um Bilddaten, welche von der Kamera 132 erzeugt werden, auszuwerten. Auf diese Weise kann beispielsweise erkannt werden, wenn Testfelder der Testelemente 116 von einer vorgegebenen Form abweichen, beispielsweise in ihrer Form und/oder Farbe. Auf diese Weise können beispielsweise Fehler, welche in der Produktionsvorrichtung 122 aufgetreten sind, erkannt werden. Auch andere Arten von Fehlern sind prinzipiell detektierbar, sowie andere Arten von Testvorrichtungen 130, beispielsweise Transmissionsmessungen, Reflexionsmessungen, Fluoreszenzmessungen, Widerstandsmessungen, Impedanzmessungen oder Kombinationen dieser und/oder anderer Messungen.

Die Testvorrichtung 130 und/oder die zentrale Steuereinheit 124 können beispielsweise derart ausgestaltet sein, dass jedem einzelnen Testfeld der Testelemente 116 eine Information zugeordnet wird. Im einfachsten Fall kann diese Information ein 1-Bit-Wert sein, welcher beispielsweise für jedes einzelne Testfeld in ein Schieberegister des Datenspeichers 128 eingetragen wird. Auf diese Weise laufen die Informationen mit den Testelementen virtuell in Durchlaufrichtung 120 mit. Auch andere Arten der Informationszuordnung sind jedoch denkbar. Im Folgenden sei davon ausgegangen, dass ein 1-Bit-Fehlerinformationswert abgespeichert wird, bei welchem beispielsweise eine "0" für "fehlerfrei" und eine "1" für "fehlerhaft" steht. Wie oben ausführlich beschrieben, sind jedoch auch andere Ausgestaltungen der Fehlerinformationen möglich.

Nach der Testvorrichtung 130 in Figur 1 durchlaufen die Testelemente 116 eine Markierungsvorrichtung 136, welche in Figur 1 ebenfalls lediglich symbolisch dargestellt ist. Entsprechend dem Ergebnis der vorangehend beschriebenen Fehlerprüfung in der Testvorrichtung 130 und den beispielsweise dementsprechend in dem Datenspeicher 128 abgelegten Fehlerinformationen für jedes Testelement 116 und/oder für jedes Testfeld der Testelemente 116 werden die Testelemente 116 in dieser Markierungsvorrichtung 136 markiert. Die Markierung ist insbesondere derart ausgestaltet, dass sich aus dieser die Fehlerinformation rekonstruieren lässt. Dabei ist es beispielsweisemöglich, jedes Testelement 116 insgesamt zu markieren und/oder jedes Testfeld der Testelemente 116 einzeln. Im Folgenden sei davon ausgegangen, dass die Markierung derart erfolgt, dass fehlerhafte Testfelder markiert werden, wohingegen fehlerfreie Testfelder und/oder fehlerfreie Testelemente 116 unmarkiert bleiben. Dabei wird im vorliegenden Ausführungsbeispiel davon ausgegangen, dass die Testmaterialien der Testelemente (d.h. die Testchemie der einzelnen Testfelder der Testelemente) selbst als Informationsträger, d.h. als strahlungssensitives Material eingesetzt werden. Dies wird nachstehend näher erläutert.

Die Markierungsvorrichtung 136 weist eine Strahlungsquelle 138 in Form einer Lichterzeugereinheit 140 auf. Diese Lichterzeugereinheit 140 ist modular aufgebaut und weist beispielsweise einen Schaltschrank 142 mit einer Energieversorgung in Form einer Stromversorgungseinheit 168 und eine Vielzahl von modularen Einzellichtquellen 144 auf. Diese Einzellichtquellen 144 sind in Figur 1 symbolisch als Leuchtdioden dargestellt, wobei vorzugsweise UV-Leuchtdioden eingesetzt werden.

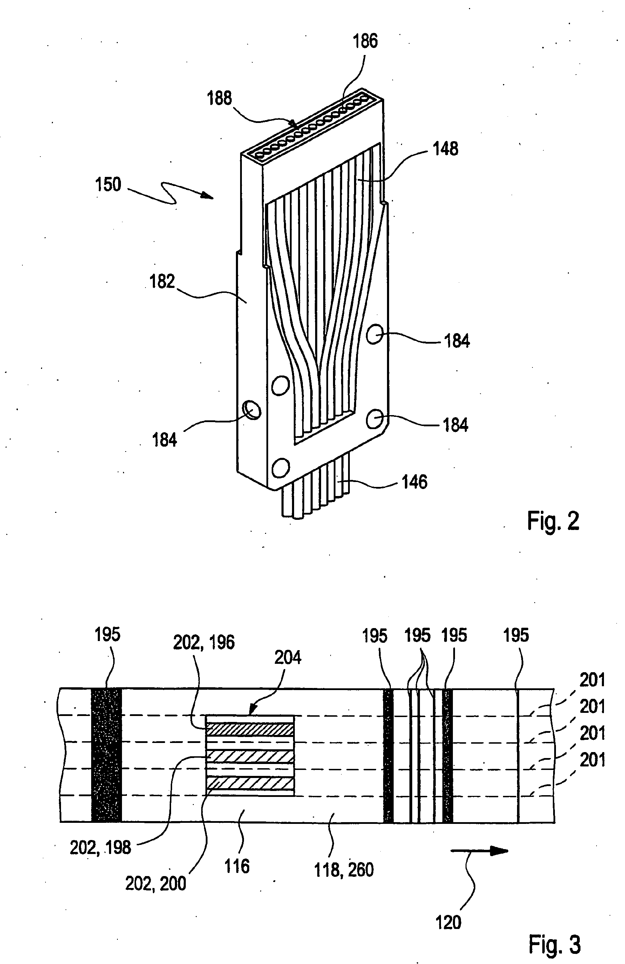

Die Strahlungsquelle 138 ist in diesem Ausführungsbeispiel vorzugsweise über ein Faserbündel 146 mit einer Vielzahl von Kunststofffaserlichtleitern 148 mit mindestens einem Querschnittswandler 150 verbunden. Dieser mindestens eine Querschnittswandler 150, welcher nachstehend anhand Figur 2 in einer möglichen Ausführungsform näher erläutert wird, ist in einer Applikationsposition 152 vorzugsweise oberhalb der Testelemente 116 angeordnet. Die Testelemente 116 werden vorzugsweise in dieser Applikationsposition in einem Führungstisch 154 geführt, wobei beispielsweise mehrere (z.B. fünf Bänder nach dem Schneiden in der Schneidevorrichtung 125) Bänder parallel durch die Applikationsposition 152 geführt werden können. Der Führungstisch 154 kann insbesondere dafür sorgen, dass die Bänder der Testelemente 116 exakt positioniert sind, insbesondere exakt zum Querschnittswandler 150. Auf diese Weise können, entsprechend der beabsichtigten Markierung der Testelemente 116 in der Markierungsvorrichtung 136, die Testelemente 116 mit einer Strahlung 156 (welche in Figur 1 lediglich symbolisch angedeutet ist) beaufschlagt werden. Die Steuerung dieser Strahlungsbeaufschlagung kann insbesondere wiederum durch die zentrale Steuereinheit 124 erfolgen, beispielsweise entsprechend den im Schieberegister des Datenspeichers 128 abgelegten Informationen für jedes einzelne Testelement 116 und/oder für jedes einzelne Testfeld der Testelemente 116.

Nach Durchlaufen der Applikationsposition 152 sind die Testelemente 116 prinzipiell einsatzbereit. Optional ist in Figur 1 eine Kontrollvorrichtung 157 vorgesehen, welche kontrolliert, ob in der Markierungsvorrichtung 136 die Markierungen korrekt aufgebracht wurden. Beispielsweise kann diese Kontrollvorrichtung 157für jedes Band eine separate Photodiode oder einen anderen Detektor umfassen, welche die Markierungen überprüft. Diese Information kann beispielsweise in der zentralen Steuereinheit ausgewertet werden, wo beispielsweise die erkannten Markierungen mit den im Datenspeicher 128, insbesondere im Schieberegister, hinterlegten Sollinformationen verglichen werden. Wird hierbei eine fehlerhafte Markierung festgestellt, so kann beispielsweise eine Warnung ausgegeben werden oder das jeweilige Testelement 116 aussortiert werden.

Weiterhin können die Testelemente 116 optional noch weiteren Verarbeitungsschritten unterzogen werden, beispielsweise einer weiteren, zumindest teilweisen Schichtung, einem Aufbringen von Schutzmaterialien oder Ähnlichem. Wie beschrieben, können sich zu diesem Zweck beispielsweise weitere Produktionsvorrichtungen 122 in Durchlaufrichtung 120 der Applikationsposition 152 nachgeordnet anschließen.

Wie eingangs beschrieben, existieren zahlreiche verschiedene Ausführungsformen von Testelementen. In Figur 1 sind daher, symbolisch und lediglich stellvertretend für die zahlreichen verschiedenen Möglichkeiten der Ausgestaltung von Testelementen, die im Rahmen der vorliegenden Erfindung eingesetzt werden können, in Form einer "Verzweigung" zwei Möglichkeiten dargestellt. So kann die Bandware der Testelemente 116 als "Vorstufe" beispielsweise in einer Vereinzelungsvorrichtung 158, welche in Figur 1 ebenfalls lediglich symbolisch dargestellt ist, zu einzelnen Testelementen 112 vereinzelt werden. An diese Vereinzelungsvorrichtung kann sich eine Sortiervorrichtung 160 anschließen, in welcher die vereinzelten Testelemente 112 entsprechend ihrer in der Applikationsposition 152 aufgebrachten Markierung in fehlerfreie und fehlerhafte Testelemente unterschieden werden kann. Wie oben beschrieben, können jedoch auch Sortierungen in mehrere Klassen anstelle einer reinen digitalen Sortierung erfolgen. An die Sortiervorrichtung 160 kann sich dann eine Verpackungsvorrichtung 162, anschließen, in welcher die Testelemente 112 beispielsweise kassettiert, magaziniert und/oder mit einer Umverpackung und/oder einer Blisterverpackung versehen werden. Das Ergebnis sind, wie in Figur 1 symbolisch dargestellt, die fertigen Testelementbehältnisse 164.

Alternativ zu der Vereinzelung und der Herstellung einzelner Testelemente 112 können die Testelemente auch als bandförmige Testelemente 114 ausgestaltet sein. Zu diesem Zweck können beispielsweise Abschnitte der bandwaren-förmigen Testelemente 116 geschnitten und in einer Kassettierungsvorrichtung 166 zu Bandkassetten (in Figur 1 symbolisch angedeutet) verarbeitet werden. Um auch aus bandförmigen Testelementen 114 fehlerhafte Testelemente (d.h. einzelne, für einen einzelnen Test geeignete Testelemente oder auch für mehrere Tests geeignete Testelemente) aussortieren zu können, kann optional der Kassettierungsvorrichtung 166 wiederum eine Sortiervorrichtung 160 vorgeschaltet sein. In dieser Sortiervorrichtung 160 können wiederum Fehlerinformationen aus den Markierungen der einzelnen Testelemente bzw. Testelementabschnitte (beispielsweise aus den Abschnitten, welche für jeweils einen einzelnen Tests geeignet sind) ausgelesen werden. Wird erkannt, dass ein Testelement 114 oder ein Abschnitt dieses Testelements 114 fehlerhaft ist, so kann dieser Abschnitt beispielsweise ausgeschnitten werden. Um dennoch anschließend ein durchgängiges Band kassettieren zu können, kann der Sortiervorrichtung 160 entsprechend eine Schneide- und Splicingvorrichtung 167 zugeordnet sein, welche in dem Beispiel der Figur 1 der Sortiervorrichtung 160 nachgeordnet ist. Auch andere Anordnungen sind jedoch möglich, beispielsweise Anordnungen, bei denen die Schneide- und Splicingvorrichtung 167 Bestandteil der Sortiervorrichtung 160 ist. In der Schneide- und Splicingvorrichtung 167 werden Bandabschnitte, die als fehlerhaft markiert sind, ausgeschnitten, entnommen, und die Enden des übrigen Bandes wieder verbunden ("Splicing"), beispielsweise durch Kleben. Diese Technik ist beispielsweise aus der traditionellen Kinofilmtechnik bekannt.

In diesem Zusammenhang wird, angesichts der vielen verschiedenen Möglichkeiten der technischen Ausgestaltung von Testelementen, darauf hingewiesen, dass der Begriff des "Testelements" im Rahmen der gesamten Erfindung weit zu fassen ist. Es soll sich um Elemente handeln, welche mindestens ein Testfeld aufweisen, welches zum qualitativen oder quantitativen Nachweis des mindestens einen Analyten geeignet ist. Als Testelement kann beispielsweise ein einzelnes, streifenförmiges Testelement 112 verwendet werden, mit einem einzelnen oder auch mehreren Testfeldern. Beispielsweise können mehrere Testfelder für jeweils einen bestimmten Analyten vorgesehen sein. Alternativ kann, wie oben ebenfalls dargelegt, auch Bandware als Testelemente verwendet werden, so dass in diesem Fall bandförmige Testelemente 114 vorliegen. In diesem Fall können die gesamten Testelementbänder der bandförmigen Testelemente 114 als ein Testelement bezeichnet werden, oder es können einzelne Testabschnitte auf diesen bandförmigen Testelemente 114, beispielsweise Testabschnitte mit jeweils einem Testfeld, als ein Testelement bezeichnet werden. Im Folgenden wird, ohne Beschränkung der möglichen weiteren Bedeutungen und abweichender Nomenklatur, letzteres angenommen, so dass bei Bandware ein einzelner Testabschnitt für einen Test als Testelement 114 bezeichnet wird.

Die Lichterzeugereinheiten 140 Markierungsvorrichtung 136 bzw. die modular aufgebauten Einzellichtquellen 144 können insbesondere derart ausgestaltet sein, dass mehrere Einzellichtquellen 144 in einem Schaltschrank 142 zusammengefasst sind, wobei in diesem Ausführungsbeispiel jeweils fünf Einzellichtquellen 144 in einer Reihe eine Stromversorgungseinheit 168 zugeordnet ist.

Die Einzellichtquellen 144 sind beispielsweise jeweils von einem Einschubgehäuse umschlossen, welches einen Einschub in den Schaltschrank 142 ermöglicht. An der Rückseite des Einschubgehäuses kann jeweils ein Steckverbinder vorgesehen sein, über welchen die modularen Einzellichtquellen 144 mit Energie versorgt werden können. Zu dem kann über diese Steckverbinder ein Datenaustausch erfolgen, so dass beispielsweise die Einzellichtquellen 144 (bzw. einzelne, in diesen Einzellichtquellen 144 aufgenommene Strahlungsquellen) gezielt angesteuert werden können, um das Markieren in der Markierungsvorrichtung 136 gezielt zu steuern. Beispielsweise kann diese Steuerung wiederum über zentrale Steuereinheit 124 erfolgen.

In den Einzellichtquellen 144 können dabei jeweils Elektronikboards aufgenommen sein, welche beispielsweise eine oder mehrere bestückte Platinen umfassen können. Auf diesen Elektronikboards kann jeweils eine Mehrzahl von Leuchtdioden, insbesondere UV-Leuchtdioden 176, aufgenommen sein, welche vorzugsweise einzeln angesteuert werden können. Bevorzugt kann es sich bei diesen UV-Leuchtdioden 176 um Leuchtdioden mit einer Wellenlänge im Bereich von ca. 250 nm bis 400 nm, insbesondere im Bereich zwischen 350 und 380 nm handeln, welche vorzugsweise eine Leistung von 50 mW bis 500 mW, besonders bevorzugt im Bereich von 100 mW bis 200 mW aufweisen. Insofern ist der Ausdruck "Einzellichtquelle" 144 nicht dahingehend zu verstehen, dass diese Einzellichtquellen 144 jeweils lediglich eine einzelne Strahlungsquelle umfassen, sondern es können auch mehrere Strahlungsquellen vorgesehen sein, wie in diesem Ausführungsbeispiel in Form von UV-Leuchtdioden 176.

Dabei kann beispielsweise pro Elektronikboard eine Anzahl von fünf UV-Leuchtdioden 176 vorgesehen sein. Zur Lichtleitung kann beispielsweise oberhalb der UV-Leuchtdioden 176 eine Koppelplatte angeordnet sein, in welcher jeweils einzelne Kunststofffaserlichtleiter 148 mit ihren Einkoppelenden fixiert sind. Diese Fixierung kann beispielsweise durch Einkleben, durch Einspannen oder durch eine Kombination von Fixierungstechniken erfolgen. Auch andere Techniken sind möglich. Die Fixierung erfolgt dabei derart, dass jeweils ein Einkoppelende der Kunststofffaserlichtleiter 148 oberhalb einer UV-Leuchtdiode 176 angeordnet ist, so dass jeweils das Licht dieser UV-Leuchtdiode 176 in einen Kunststofffaserlichtleiter 148 eingekoppelt wird. Für die Einkopplung ist diese einfache Zuordnung in der Regel ausreichend, es können jedoch auch komplexere Einkoppeloptiken vorgesehen sein, wie beispielsweise Linsensysteme, insbesondere Mikrolinsensysteme, oder ähnliche Einkoppelvorrichtungen.

Die Kunststofffaserlichtleiter 148 werden anschließend zu Faserbündeln 146 zusammengefasst, welche beispielsweise über Zugentlastungen aus dem Einschubgehäuse der Einzellichtquellen 144 herausgeführt werden können. Anschließend können mehrere der Faserbündel 146 zu übergeordneten Faserbündeln 146 zusammengefasst werden, um beispielsweise als gemeinsames Faserbündel 146 zur Applikationsposition 152 in Figur 1 (wobei sinngemäß auch mehrere Applikationspositionen 152 vorgesehen sein können) geführt zu werden.

In Figur 2 ist ein mögliches Ausführungsbeispiel eines Querschnittswandlers 150 in perspektivischer Darstellung gezeigt. Der Querschnittswandler 150 umfasst einen Rahmen 182, in welchem beispielsweise eine Mehrzahl von Öffnungen 184 vorgesehen sein können, welche insbesondere ein Verstiften, Verschrauben oder anderweitiges Fixieren des Querschnittswandlers 150 ermöglichen. Diese Öffnungen 184, welche beispielsweise als Verstiftungsbohrungen oder Stiftbohrungen ausgeführt sein können, sind in Figur 2 lediglich schematisch dargestellt und können auch der jeweiligen Verstiftungssituation oder anderen Montagetechniken angepasst und andersartig ausgestaltet sein. Auf diese Weise können mehrere Querschnittswandler 150, beispielsweise durch Verstiften oder Verschrauben, zu Querschnittswandlermodulen zusammengefasst und in der Applikationsposition 152 fixiert werden. Die Rahmen 182 können beispielsweise aus Aluminium, Edelstahl, Kunststoff und/oder anderen Materialien gefertigt sein.

Die Querschnittswandler 150 sind dabei derart ausgestaltet, dass diese ein ankommendes Faserbündel 146 (in Figur 2 unten) in einzelne Kunststofffaserlichtleiter 148 aufspalten. Dabei werden die Auskoppel-Faserenden der einzelnen Kunststofffaserlichtleiter 148 (in Figur 2 oben zu einem gewünschten Muster 188 angeordnet und in dieser Weise fixiert. Wiederum kann diese Fixierung beispielsweise durch ein Einspannen, Einkleben oder durch eine Art der Fixierung erfolgen. Bei dem in Figur 2 dargestellten, bevorzugten Ausführungsbeispiel, weist das Muster 188 ein Linienmuster auf, bei welchem in diesem Ausführungsbeispiel fünfzehn Faserenden 186 vorzugsweise zumindest näherungsweise äquidistant zu einer Linie angeordnet sind. Da vorzugsweise jeweils zwei oder mehr derartiger Querschnittswandler 150 in einem Modul in Durchlaufrichtung 120 hintereinander angeordnet sind, ergibt sich somit insgesamt jeweils ein Linienmuster mit dreißig oder mehr in Reihe angeordneten Faserenden 186. Jedes Faserende 186 kann beispielsweise einen Durchmesser von ca. 1 mm bis 3 mm aufweisen. Eine derartige Linie emittiert beispielsweise insgesamt eine UV-Lichtleistung von ca. 500 mW bis 1500 mW, beispielsweise ca. 1000 mW.

Wie oben beschrieben, können jedoch auch andere Arten von Mustern 188 verwendet werden, beispielsweise Matrixmuster mit beispielsweise rechteckförmiger Matrix. Auch andere Ausgestaltungen sind möglich. Insbesondere können auch andere Arten von Fasern anstelle der Kunststofffaserlichtleiter 148 eingesetzt werden, beispielsweise Glasfasern. Auch kann, anstelle der in Figur 2 dargestellten einfachen Auskopplung aus den Faserenden 186 eine zusätzliche Strahlformungsoptik vorgesehen sein, beispielsweise mit einer oder mehreren gemeinsamen Linsen für alle Faserenden 186, oder mit individuellen Linsen für die Faserenden 186. Auf diese Weise können auch die Strahlquerschnitte weiter angepasst werden.

Bei der Anordnung gemäß Figur 1 können beispielsweise mehrere Querschnittswandler 150 in der Applikationsposition 152 zu einem Applikationsmodul 190 zusammengefasst sein. Dabei sind vorzugsweise mehrere Querschnittswandler 150 über die oben in Figur 2 dargestellten Öffnungen 184 mit Stiften verstiftet sind und/oder verschraubt. Die Öffnungen 184 können beispielsweise als Bohrungen und/oder Stiftbohrungen und/oder Gewindebohrungen ausgestaltet sein. Insbesondere kann auch ein Klemmrahmen vorgesehen sein, um die einzelnen Module der Querschnittswandler 150 aufzunehmen. In der Darstellung nach Figur 1 kann beispielsweise jeder der Querschnittswandler 150 dem Ausführungsbeispiel gemäß Figur 2 entsprechen, wobei die Querschnittswandler 150 jedoch im Vergleich zu Figur 2 umgedreht sind, so dass die Faserenden 186 (nicht erkennbar in Figur 1) in Figur 1 nach unten weisen. Dabei sind vorzugsweise (was in Figur 1 nicht dargestellt ist) jeweils zwei derartige Querschnittswandler 150 seriell hintereinander in Durchlaufrichtung 120 angeordnet, und vorzugsweise jeweils fünf derartiger Querschnittswandler-Paare nebeneinander, senkrecht zur Durchlaufrichtung 120.

Der in Figur 1 schematisch dargestellte Führungstisch 154 umfasst beispielsweise fünf rechteckförmige Führungsnuten, welche von ihrer Dimensionierung her jeweils auf die Bandware der Testelemente 116 abgestimmt sind. Diese Dimensionierung kann beispielsweise derart gestaltet sein, dass diese Führungsnuten in ihrer Breite den Einzelstreifen entsprechen, welche nach der Schneidevorrichtung 125 (in welcher breite Teststreifen zu beispielsweise drei, fünf oder einer anderen Anzahl von schmaleren Streifen längs geschnitten werden) entstehen und jeweils den Führungsnuten zugeführt werden. Auch eine Gesamtpositionierung ungeschnittener Testelemente 116 durch den Führungstisch 154 ist jedoch prinzipiell möglich.

Auf diese Weise lassen sich durch den Führungstisch 154 und dessen Führungsnuten die bandförmigen Testelemente 116 präzise führen, so dass diese Testelemente 116 bzw. darauf angeordnete Testfelder und/oder andere Arten von strahlungssensitiven Materialien (z.B. Markierungsfelder) exakt zu den Faserenden 186 positioniert werden können. Da beispielsweise in dem Schieberegister des Datenspeichers 128 in Figur 1 Informationen enthalten sind, welche Testelemente bzw. Testfelder oder Markierungsfelder sich gerade in der Applikationsposition 152 befinden, können die Einzellichtquellen 144 bzw. die darin aufgenommenen Leuchtdioden 176 auf diese Weise gezielt geschaltet werden, um gezielt bestimmte Testelemente 116, bestimmte Testfelder und/oder bestimmte Markierungsfelder zu markieren. Auch können, alternativ oder zusätzlich, hier wie in anderen möglichen Ausführungsformen der Erfindung Positionierungsmarkierungen auf den Testelementen 116 vorgesehen sein, welche eine Positionierung und/oder Identifikation der Testelemente 116 für eine Markierung zusätzlich erleichtern.

In Figur 3 ist ein Ausführungsbeispiel eines Testelements 116 dargestellt. Das Testelement 116 ist in diesem Beispiel als Bandware ausgestaltet, wobei lediglich ein Testabschnitt des Bandes gezeigt ist. Das Band des Testelements 116 ist in diesem Ausführungsbeispiel mit einer Vielzahl von Positionierungsmarkierungen 195 versehen, welche auf das Endlosband 118 aufgedruckt sind, beispielsweise in einem Siebdruckverfahren. Diese Positionierungsmarkierungen 195 können bei der Herstellung der Testelemente 116 der Positionierung der Bandware dienen und/oder können später, beim Einsatz der Testelemente 116 in einem Analysegerät, der korrekten Positionierung der Testelemente für eine Probenapplikation und/oder für eine Auswertung der Tests dienen.

Das Ausführungsbeispiel in Figur 3 zeigt ein Testelement 116 vor dem Durchlaufen einer Schneidevorrichtung 125 (siehe Figur 1). Dies bedeutet, dass es sich in dem dargestellten Fall noch um ein breites, ungeschnittenes Rohband handelt, bei welchem jeweils drei einzelne (in diesem Fall rechteckige) Testfelder 196, 198 und 200 parallel zueinander auf dem Endlosband 118 angeordnet sind und in diesem Zustand noch ein Gesamttestfeld 204 bilden. Dieses ungeschnittene Band kann anschließend noch in der Schneidevorrichtung 125 geschnitten werden, beispielsweise entlang der in Figur 3 angedeuteten Schnittlinien 201, so dass schließlich die eigentlichen bandförmigen Testelemente 116 entstehen, bei welchen jeweils nur eines der Testfelder 196, 198, 200 nebeneinander angeordnet ist. Die Randstreifen ohne Testfelder können dabei verworfen werden. Alternativ könnte das in Figur 3 dargestellte Band jedoch auch ungeschnitten eingesetzt werden, so dass ein Testelement 116 drei Testfelder 196, 198, 200 nebeneinander aufweist. Diese könnten beispielsweise für eine Mittelwertbildung verwendet werden.

Die Testfelder 196, 198 und 200 umfassen beispielsweise eine Nachweischemie, welche bei Applikation einer flüssigen Probe, beispielsweise einer Blutprobe, einen Farbumschlag durchführt, entsprechend der Anwesenheit eines Analyten, zum Beispiel Blutglukose. Diese Nachweischemie wird im vorliegenden Ausführungsbeispiel als strahlungssensitives Material 202 genutzt, welches als Informationsträger für die Fehlerinformation verwendet wird. Wie oben dargestellt, könnten jedoch in diesem oder in anderen Ausführungsbeispielen auch separate Markierungsfelder eingesetzt werden, welche, unabhängig von der Testchemie, strahlungssensitive Materialien aufweisen.

Das in Figur 3 dargestellte Ausführungsbeispiel eines Testelements 116 hat bereits die Applikationsposition 152 durchlaufen. Dabei wurde mittels der Strahlungsquelle 138 gezielt das obere Testfeld 196 in Figur 3 bestrahlt, was in einer sichtbaren Farbveränderung dieses Testfeldes 196 gegenüber den unbestrahlten Testfeldern 198, 200 resultiert. Auf diese Weise lässt sich beispielsweise das obere Testfeld 196 gezielt unbrauchbar für eine Analyse machen. Diese Farbveränderung kann beispielsweise photometrisch (z.B. durch eine Reflexions-, Transmissions- oder Farbmessung oder anderer Arten von Messungen) erkannt werden. Es sei darauf hingewiesen, dass das in Figur 3 dargestellte Testelement 116 somit nicht in der in Figur 1 gezeigten Herstellungsvorrichtung 110 hergestellt wurde, da die Testelemente 116 in Figur 1 vor Erreichen der Markierungsvorrichtung 136 bereits in der Schneidevorrichtung 125 geschnitten werden, im Gegensatz zu dem ungeschnittenen und dennoch markierten Testelement 116 in Figur 3. Das Beispiel in Figur 3 dient jedoch lediglich zur Verdeutlichung des Prinzips der Erfindung. Es ist auch eine Markierung nach dem Schneiden möglich, analog zu Figur 1.

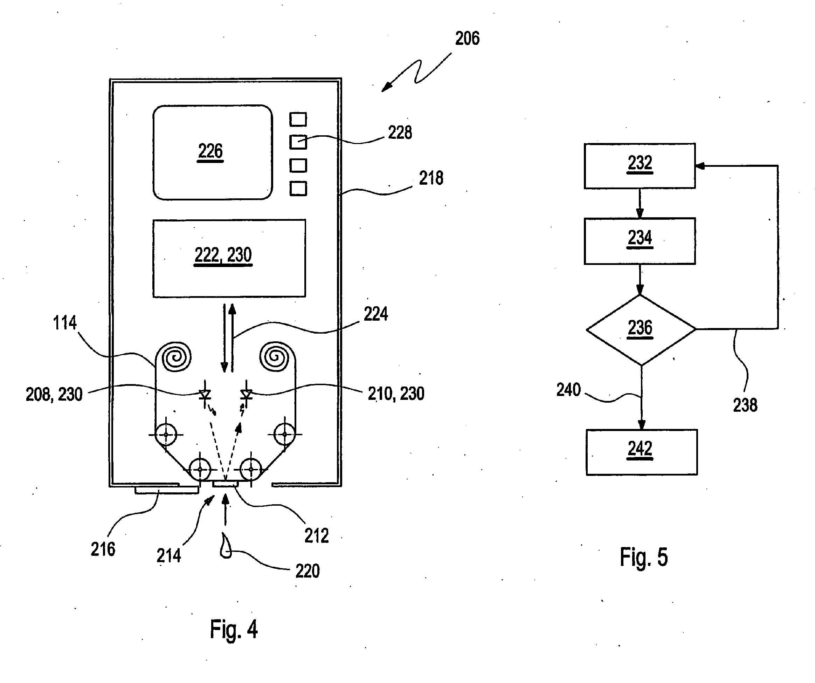

In Figur 4 ist ein Ausführungsbeispiel eines erfindungsgemäßen analytischen Testgeräts 206 symbolisch dargestellt, welches mit erfindungsgemäß markierten Testelementen 114 arbeitet. Daneben ist in Figur 5 ein mögliches Verfahren zum Nachweis mindestens eines Analyten in einer Probe dargestellt, welches insbesondere in Zusammenhang mit dem in Figur 4 dargestellten analytischen Testgerät realisiert werden kann, welches jedoch auch unabhängig hiervon zum Einsatz kommen kann.

Es sei darauf hingewiesen, dass im Idealfall, welcher auch den Normalfall darstellt, die Herstellung der Testelemente 114 so erfolgt, dass keine als fehlerhaft markierten Testelemente 114 in Umlauf geraten. Insofern stellt das nachfolgend beschriebene analytische Testgerät 206 oder ein erfindungsgemäßes analytisches Testgerät 206 gemäß einer anderen Ausführungsform der Erfindung, lediglich eine zusätzliche Sicherheit bereit, dass, sollten trotz eines Aussortierens fehlerhafter Testelemente 114 bei der Herstellung derartige als fehlerhaft markierte Testelemente 114 in Umlauf geraten, diese nicht für Tests verwendet werden. Alternativ könnte jedoch, was weniger bevorzugt ist, mittels des analytischen Testgeräts 206 eine Selektion auch erst beim Testen erfolgen, so dass erst beim Testen als fehlerhaft markierte Testelemente 114 aussortiert, d.h. nicht verwendet werden. Dies hätte den Nachteil, dass in diesem Fall unter Umständen eine geringere Anzahl an Testelementen 114 zur Verfügung stünde, was aber, insbesondere bei einer Vielzahl von Einzeltestelementen 114 auf einem Band, möglicherweise durch eine Überzahl von Testelementen 114 (d.h. einer zusätzlichen Anzahl oberhalb einer Nennanzahl an Einzeltestelementen 114) ausgeglichen werden könnte.

Das analytische Testgerät 206 weist in diesem Ausführungsbeispiel beispielsweise ein bandförmiges Testelement 114 auf, z.B. ein in einer Bandkassette aufgenommenes Testelement 114. Alternativ oder zusätzlich können jedoch auch andere Arten von Testelementen vorgesehen sein, beispielsweise streifenförmige Testelemente, beispielsweise in einem Stangenmagazin, einem Trommelmagazin, einem Scheibenmagazin oder in einer anderen Art von Magazin.

Das analytische Testgerät 206 weist in diesem Ausführungsbeispiel eine optische Anregungsvorrichtung 208 und eine optische Detektionsvorrichtung 210 auf, welche in Figur 4 lediglich symbolisch angedeutet sind. Mittels dieser Anregungsvorrichtung 208, welche beispielsweise eine oder mehrere Lichtquellen umfassen kann, und der Detektionsvorrichtung 210, welche beispielsweise eine oder mehrere Fotodioden umfassen kann, können beispielsweise ein oder mehrere Testfelder 212 auf dem Testelement 114 in einer Testposition 214 auf Analyt-bedingte Farbänderungen untersucht werden. Das analytische Testgerät 206 weist in diesem Beispiel vorzugsweise einen Verschluss 216 in einem Gehäuse 218 des analytischen Testgerätes 206 auf. Dieser Verschluss 216 ergibt die Testposition 214 bzw. ein in dieser Testposition 214 befindliches Testfeld 212 für die Applikation einer Probe 220, wobei es sich in diesem Ausführungsbeispiel vorzugsweise um eine flüssige Probe handelt, frei.

Das analytische Testgerät 206 weist weiterhin eine Ansteuer- und Auswerteeinheit 222 auf. Diese Ansteuer- und Auswerteeinheit 222 kann beispielsweise eine oder mehrere Mikrocomputer umfassen und eingerichtet sein, um die Anregungsvorrichtung 208 und/oder die Detektionsvorrichtung 210 anzusteuern. Weiterhin kann ein Transport des bandförmigen Testelements 114 gesteuert werden, so dass jeweils eine Beförderung eines Testfeldes 212 in Testposition 214 gesteuert werden kann. Diese Steuerungen und umgekehrt ein Datenaustausch sind in Figur 4 symbolisch durch den Doppel-Pfeil 224 angedeutet.

Daneben weist das analytische Testgerät 206 vorzugsweise Anzeigemittel, insbesondere ein Display 226 sowie Bedienelemente 228 auf. Auf diese Weise lassen sich die Funktionen des analytischen Testgeräts 206 steuern, und Messinformationen lassen sich ausgeben.

Im Normalbetrieb, d.h. einem dem Stand der Technik bekannten Betrieb, wird, gesteuert durch die Ansteuer- und Auswerteeinheit 222, ein bestimmtes Testfeld 212 in die Testposition 214 gefahren, der Verschluss 216 wird freigegeben, und die Applikation der Probe 220 wird ermöglicht. Anschließend erfolgt durch die Anregungsvorrichtung 208 und die Detektionsvorrichtung 210 eine optische Auswertung des Testfeldes 212, so dass beispielsweise eine Analytkonzentration insbesondere eine Blutzuckerkonzentration bestimmt werden kann. Diese kann beispielsweise auf dem Display 226 ausgegeben werden.

Erfindungsgemäß weist das analytische Testgerät 206 jedoch in dem in Figur 4 dargestellten Ausführungsbeispiel darüber hinaus eine Abfragevorrichtung 230 auf, welche eingerichtet ist, um Markierungen, welche beispielsweise gemäß dem oben beschriebenen Verfahren auch auf das Testelement 114 und/oder das Testfeld 212 aufgebracht wurden, zu erkennen und auszuwerten. Diese Abfragevorrichtung 230 nutzt in diesem in Figur 4 dargestellten Ausführungsbeispiel die Anregungsvorrichtung 208 und die Detektionsvorrichtung 210 sowie einen entsprechenden, beispielsweise softwaretechnisch in der Ansteuer-und Auswerteeinheit 222 implementierten Abfragealgorithmus. Alternativ könnte die Abfragevorrichtung 230 jedoch auch als separate, von der Anregungsvorrichtung 208 und der Detektionsvorrichtung 210 getrennte Vorrichtung implementiert sein, beispielsweise mittels einer separaten Abfrage-Anregungsvorrichtung und/oder einer separaten Abfrage-Detektionsvorrichtung (in Figur 4 nicht dargestellt). Auf diese Weise können markierte Testelemente 114 und/oder markierte Testfelder 212 erkannt werden, beispielsweise in dem eine Färbung der Testfelder 212 detektiert wird.

Bei dem vorgeschlagenen Verfahren wird zunächst ein bestimmtes Testelement 114 und/oder ein bestimmtes Testfeld 212 in der Testposition 214 bereitgestellt. Dies ist in Figur 5 symbolisch mit der Bezugsziffer 232 bezeichnet. Anschließend wird mittels der Abfragevorrichtung 230 abgefragt, ob das Testfeld 212 und/oder das Testelement 114 mit einer Markierung versehen ist, und/oder es wird die in der Markierung enthaltene Fehlerinformation ausgelesen (Schritt 234). Diese Information kann beispielsweise in der Ansteuer- und Auswerteeinheit 222 ausgewertet werden. Anschließend kann ein Entscheidungsschritt 236 durchgeführt werden, bei welchem entschieden wird, ob das Testfeld 212 und/oder das Testelement 114 entsprechend der ausgelesenen Fehlerinformation fehlerhaft ist (Zweig 238 oder fehlerfrei (Zweig 240 in Figur 5). Wird das Testfeld 212 und/oder das Testelement 114 als fehlerhaft erkannt, so kann optional eine Warnung an einen Benutzer ausgegeben werden, beispielsweise in Form einer visuellen Warnung (z.B. auf dem Display 226) und/oder einer akustischen Warnung. Alternativ oder zusätzlich kann, wie in Figur 5 dargestellt, zum Schritt 232 zurückgekehrt werden, wobei ein neues Testfeld 212 und/oder Testelement 114 bereitgestellt wird. Bei der Anordnung gemäß Figur 4 kann dabei z.B. ein frisches, bislang unbenutztes Testfeld 212 in die Testposition 214 gefahren werden. Beispielsweise können bei der Anordnung in Figur 4 die bislang beschriebenen Verfahrensschritte so durchgeführt werden, dass während der Durchführung dieser Verfahrensschritte der Verschluss 216 geschlossen ist, so dass eine Applikation der Probe 220 bislang nicht möglich ist.

Wird hingegen in Schritt 236 erkannt, dass das Testfeld fehlerfrei ist, so kann anschließend eine Messung 242 erfolgen, wobei der Nachweis mindestens eines Analyten in der Probe 220 erfolgt. Beispielsweise kann im Rahmen dieser Messung 242 in Figur 4 der Verschluss 216 freigegeben werden und/oder es können die Anregungsvorrichtung 208 und die Detektionsvorrichtung 210 in betrieb gesetzt werden, beispielsweise um eine Analyt-bedingte Farbänderung durchzuführen.

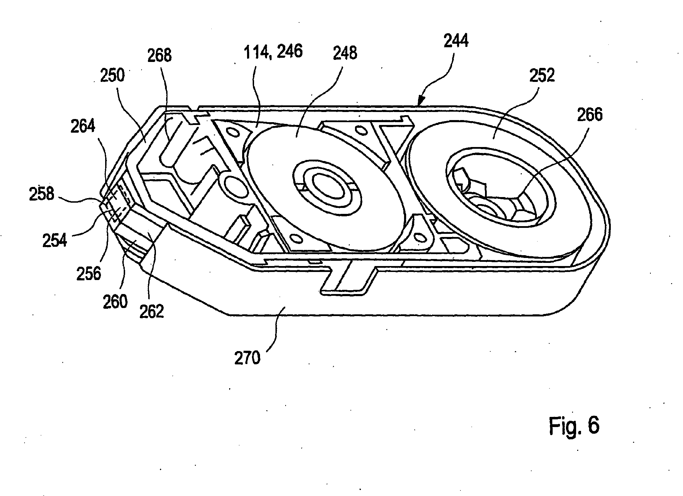

In Figur 6 ist ein Ausführungsbeispiel einer möglichen Bandkassette 244 dargestellt, welche beispielsweise in dem in Figur 4 dargestellten analytischen Testgerät 206 eingesetzt werden kann, und welches beispielsweise die Durchführung einer Vielzahl von Glukoseuntersuchungen an vor Ort vom Patienten selbst gewonnenen flüssigen Proben 220 (beispielsweise Blutproben) ermöglicht. Zu diesem Zweck umfasst die Bandkassette 244 ein Testelement 114 in Form eines analytischen Testbandes 246. Das analytische Testband 246 ist von einer Vorratsspule 248 abziehbar und über eine Bandführung 250 auf eine Aufwickelspule 252 aufwickelbar. Dabei wird ein Testbandabschnitt 254 des analytischen Testbandes 246 an einer Messstelle 256 über einem ebenen Auflagerahmen 258 plan aufgespannt, um eine vorderseitige Applikation der flüssigen Probe 220, beispielsweise in Form von Körperflüssigkeit (z.B. Blut bzw. Gewebeflüssigkeit), und eine präzise rückseitige reflektometrische Vermessung zu erlauben.

Das Testband 246 weist ein lichtdurchlässiges Trägerband 260 auf, welches beispielsweise dem Endlosband 118 in Figur 3 (in geschnittener Form) entsprechen kann. Auf der Vorderseite dieses Trägerbandes 260 sind, beispielsweise analog zur Figur 3 oben, Testfelder etikettenartig aufgebracht, welche beispielsweise den Testfeldern 196, 198 und 200 in Figur 3 entsprechen können. Diese Testfelder 262 können beispielsweise Trockenchemikalien umfassen, welche auf den Analyten (beispielsweise Glukose) in der applizierten flüssigen Probe 220 (beispielsweise Blutflüssigkeit) ansprechen und bei rückseitiger Beleuchtung zu einer messbaren Veränderung der Lichtrückstrahlung führen. Beispielsweise kann das Trägerband 260 eine 5 mm breite und etwa 10 µm dicke Folie aufweisen, auf die ein Nachweisfilm von 50 µm Dicke abschnittsweise vorderseitig aufgebracht (beispielsweise aufetikettiert) ist.

Bei einer Messung wird Messlicht über eine von dem Auflagerahmen 258 umrandete Messöffnung 264 eingestrahlt und zurückgeworfen, ohne dass innerhalb des Öffnungsbereichs Optikelemente wie Linsen, Filter oder materiell ausgefüllte Fenster vorhanden sein müssen. Die Messöffnung 264 kann jedoch bei Bedarf durch eine Blende umrandet sein (in Figur 6 nicht dargestellt). Dies erlaubt eine definierte rückseitige Fokussierung bzw. Ausrichtung einer (nicht in der Bandkassette 244 enthaltenen) optischen Messeinheit des analytischen Testgeräts 206 auf den Testbandabschnitt 254, der über der Messöffnung 264 plan freigelegt ist.

Um die Testfelder 262 sukzessive an die Messstelle 256 zu transportieren, ermöglicht ein in eine Nabe 266 der Aufwickelspule 252 eingreifender Bandantrieb des analytischen Testgeräts 206 das Vorspulen des Testbands 246. Hierbei werden durch Reibung an der Vorratsspule 248 und im Bereich der Bandführung 250 (dort insbesondere an einer Durchzugdichtung 268) Rückhaltekräfte von etwa 2 Newton erzeugt, so dass das Testband 246 ausreichend unter Zug gesetzt wird, um eine ebene Auflage auf dem Auflagerahmen 258 zu gewährleisten.

Die Bandführung 250 kann beispielsweise durch ein Spritzgussteil aus Polypropylen gebildet sein, welches zugleich einen Trägerkörper für die Spulen 248, 252 bilden kann. Zur Abdeckung der Bandführung 250 nach außen ist ein Deckelteil 270 vorgesehen, welches an einer verjüngten Schmalseitenwand einen Durchbruch für eine einfach zugängliche Freilegung des Auflagerahmens 258 aufweist.

Wie oben dargestellt, kann als Testelement 114, welches hier in kassettierter Form vorliegt, entweder das gesamte analytische Testband 246 aufgefasst werden, oder es können auch einzelne Testbandabschnitte 254 (beispielsweise Testbandabschnitte mit jeweils einem Testfeld 262) als derartige Testelemente 114 betrachtet werden. Dabei kann jeweils das Testband 246 als ganzes markiert sein, oder es kann, alternativ oder zusätzlich, auch eine Markierung einzelner Testbandabschnitte 254 mittels des oben vorgeschlagenen Verfahrens erfolgen. Wird eine separate Vorrichtung für die Auslesung von Fehlermarkierungen verwendet, so kann diese beispielsweise in die genannte optische Messeinheit des analytischen Testgeräts 206 integriert werden. Alternativ kann, wie oben beschrieben, beispielsweise auch die Anregungsvorrichtung 208 und die Detektionsvorrichtung 210 des analytischen Testgeräts 206 neben der Analysefunktion auch die Funktion der Auslesung der Fehlermarkierungen übernehmen. Auch Kombinationen dieser beiden Möglichkeiten sind denkbar, beispielsweise im Rahmen einer separaten Lichtquelle für die Auslesung der Fehlermarkierung, wobei jedoch die Detektionsvorrichtung 210 gleichzeitig auch die Aufgabe der Fehlerauslesung übernimmt.

Auf diese Weise kann, unter Verwendung der in Figur 6 dargestellten Bandkassette 244, beispielsweise das analytische Testgerät 206 derart eingerichtet sein, dass vor Durchführung einer Messung abgefragt wird, ob der gerade in der Messstelle 256 befindliche Testbandabschnitt 254 fehlerhaft ist. Ist dies der Fall, so wird durch entsprechende Betätigung der Spulen 248, 252 beispielsweise auf den nächsten Testbandabschnitt 254 weitergespult, und die Prozedur wiederholt, beispielsweise unter Verwendung des oben anhand der Figur 5 beschriebenen Verfahrens.

### Bezugszeichenliste

- 110: Herstellungsvorrichtung
- 112: Testelemente, vereinzelt
- 114: Testelemente, kassettiert
- 116: Testelemente, Bandware
- 118: Endlosband
- 120: Durchlaufrichtung
- 122: Produktionsvorrichtung
- 123: Klebestation zum Aufkleben der Testmaterialien
- 124: zentrale Steuereinheit
- 125: Schneidevorrichtung
- 126: Prozessor
- 128: Datenspeicher
- 130: Testvorrichtung
- 132: Kamera
- 134: Bilderkennungssystem
- 136: Markierungsvorrichtung
- 138: Strahlungsquelle
- 140: Lichterzeugereinheit
- 142: Schaltschrank
- 144: Einzellichtquellen
- 146: Faserbündel
- 148: Kunststofffaserlichtleiter
- 150: Querschnittswandler
- 152: Applikationsposition
- 154: Führungstisch
- 156: Strahlung
- 157: Kontrollvorrichtung
- 158: Vereinzelungsvorrichtung
- 160: Sortiervorrichtung
- 162: Verpackungsvorrichtung
- 164: fertiges Testelementbehältnis
- 166: Kassettierungsvorrichtung
- 167: Schneide- und Splicingvorrichtung
- 168: Stromversorgungseinheit
- 176: UV-Leuchtdioden
- 182: Rahmen
- 184: Öffnungen
- 186: Faserenden
- 188: Muster
- 190: Applikationsmodul
- 195: Positionierungsmarkierungen
- 196: Testfeld
- 198: Testfeld
- 200: Testfeld
- 201: Schnittlinien
- 202: strahlungssensitives Material
- 204: Gesamttestfeld
- 206: analytisches Testgerät
- 208: Anregungsvorrichtung
- 210: Detektionsvorrichtung
- 212: Testfeld
- 214: Testposition
- 216: Verschluss
- 218: Gehäuse
- 220: Probe
- 222: Ansteuer- und Auswerteeinheit
- 224: Steuerung
- 226: Display
- 228: Bedienelemente
- 230: Abfragevorrichtung
- 232: Bereitstellen eines neuen Testfeldes / Testelements
- 234: Abfrage Markierung
- 236: Testfeld / Testelement fehlerhaft?
- 238: Testfeld / Testelement fehlerhaft
- 240: Testfeld / Testelement fehlerfrei
- 242: Messung
- 244: Bandkassette
- 246: analytisches Testband
- 248: Vorratsspule
- 250: Bandführung
- 252: Aufwickelspule
- 254: Testbandabschnitt
- 256: Messstelle
- 258: Auflagerahmen
- 260: Trägerband
- 262: Testfelder
- 264: Messöffnung
- 266: Nabe
- 268: Durchzugdichtung
- 270: Deckelteil

## Patentansprüche

1. Markierungsverfahren zur Markierung von Testelementen (112, 114, 116; 246, 254), wobei die Testelemente (112, 114, 116; 246, 254) eingerichtet sind, um mindestens einen Analyten in einer Probe (220) nachzuweisen, wobei die Testelemente (112, 114, 116; 246, 254) zumindest teilweise mit einer Fehlermarkierung versehen werden, welche eine Information über eine Fehlerhaftigkeit der Testelemente (112, 114, 116; 246, 254) beinhaltet, **dadurch gekennzeichnet, dass** die Testelemente (112, 114, 116; 246, 254) mindestens ein strahlungssensitives Material (202) aufweisen, wobei die Testelemente (112, 114, 116; 246, 254) mit mindestens einer Strahlung (156) beaufschlagt werden, wobei die Strahlung (156) eingerichtet ist, um eine Markierung in Form mindestens einer optisch nachweisbaren Änderung in dem strahlungssensitiven Material (202) zu bewirken.

2. Markierungsverfahren nach dem vorhergehenden Anspruch, wobei die Strahlung (156) eine elektromagnetische Strahlung, insbesondere eine ultraviolette Strahlung, insbesondere eine Strahlung im Wellenlängenbereich zwischen 250 nm und 400 nm, vorzugsweise im Wellenlängenbereich von 350 nm bis 380 nm, umfasst.

3. Markierungsverfahren nach einem der beiden vorhergehenden Ansprüche, wobei mindestens eine der folgenden Lichtquellen zur Erzeugung der Strahlung (156) verwendet wird: eine Glühlampe; eine Gasentladungslampe; ein Laser; eine Leuchtdiode (176); eine Blitzlampe.

4. Markierungsverfahren nach einem der vorhergehenden Ansprüche, wobei die optisch nachweisbare Änderung mindestens eine der folgenden Änderungen umfasst: eine Farbänderung; eine Änderung einer Lumineszenz, insbesondere einer Fluoreszenz; eine Änderung einer Reflektivität.

5. Markierungsverfahren nach einem der vorhergehenden Ansprüche, wobei das mindestens eine strahlungssensitive Material (202) mindestens ein Testmaterial der Testelemente (112, 114, 116; 246, 254) umfasst, wobei das mindestens eine Testmaterial eingerichtet ist, um bei Anwesenheit des mindestens einen Analyten in der Probe (220) mindestens eine messbare Eigenschaft zu ändern, insbesondere eine messbare elektrische und/oder optische Eigenschaft.

6. Markicruogsverfahren nach einem der vorhergehenden Ansprüche, wobei das mindestens eine strahlungssensitive Material (202) mindestens ein Material mindestens eines von einem Testmaterial der Testelemente (112, 114, 116; 246, 254) verschiedenen Markierungsfeldes umfasst.

7. Herstellungsverfahren zur Herstellung von Testelementen (112, 114, 116; 246, 254), wobei die Testelemente (112, 114, 116; 246, 254) eingerichtet sind, um mindestens einen Analyten in einer Probe (220) nachzuweisen, wobei das Verfahren folgende Schritte umfasst:
- eine Mehrzahl der Testelemente (112, 114, 116; 246, 254) wird hergestellt;
- mindestens eines der Testelemente (112, 114, 116; 246, 254) wird einer Fehlerprüfung unterzogen, wobei festgestellt wird, ob das Testelement (112, 114, 116; 246, 254) fehlerhaft ist;
- mindestens eines der Testelemente (112, 114, 116; 246, 254) wird mit einer Fehlermarkierung versehen, welche eine Information über eine Fehlerhaftigkeit des Testelements (112, 114, 116; 246, 254) beinhaltet, wobei ein Markierungsverfahren nach einem der vorhergehenden Ansprüche verwendet wird.

8. Herstellungsverfahren nach dem vorhergehenden Anspruch, wobei in einem Aussortierungsschritt als fehlerhaft markierte Testelemente (112, 114, 116; 246, 254) aussortiert werden.

9. Herstellungsverfahren nach einem der beiden vorhergehenden Ansprüche, wobei mehrere Testelemente (112, 114, 116; 246, 254) als Bandware mit einem gemeinsamen Trägerband (118; 260) hergestellt werden.

10. Herstellungsverfahren nach einem der vorhergehenden, auf ein Herstellungsverfahren gerichteten Ansprüche, wobei nach dem Verfahrensschritt, in welchem mindestens eines der Testelemente (112, 114, 116; 246, 254) mit der Fehlermarkierung versehen wird, ein Kontrollschritt durchgeführt wird, bei welchem überprüft wird, ob die Fehlermarkierung korrekt aufgebracht wurde.

11. Markierungsvorrichtung (136) zur Markierung fehlerhafter Testelemente (112, 114, 116; 246, 254), insbesondere zur Durchführung eines Markierungsverfahrens nach einem der vorhergehenden, auf ein Markierungsverfahren gerichteten Ansprüche, wobei die Testelemente (112, 114, 116; 246, 254) eingerichtet sind, um mindestens einen Analyten in einer Probe (220) nachzuweisen, wobei die Markierungsvorrichtung (136) eingerichtet ist, um die Testelemente (112, 114, 116; 246, 254) zumindest teilweise mit einer Fehlermarkierung zu versehen, welche eine Information über eine Fehlerhaftigkeit der Testelemente (112, 114, 116; 246, 254) beinhaltet, **dadurch gekennzeichnet, dass** die Testelemente (112, 114, 116; 246, 254) mindestens ein strahlungssensitives Material (202) aufweisen, wobei die Markierungsvorrichtung (136) mindestens eine Strahlungsquelle (138) aufweist, um die Testelemente (112, 114, 116; 246, 254) mit mindestens einer Strahlung (156) zu beaufschlagen, wobei die Strahlung (156) eingerichtet ist, um eine Markierung in Form mindestens einer optisch nachweisbaren Änderung in dem strahlungssensitiven Material (202) zu bewirken.

12. Markierungsvorrichtung (136) nach dem vorhergehenden Anspruch, weiterhin aufweisend mindestens eine Testvorrichtung (130), wobei die Testvorrichtung (130) eingerichtet ist, um mindestens eines der Testelemente (112, 114, 116; 246, 254) einer Fehlerprüfung zu unterziehen, wobei die Markierungsvorrichtung (136) eingerichtet ist, um zu entscheiden, ob das Testelement (112, 114, 116; 246, 254) fehlerhaft ist.

13. Markierungsvorrichtung (136) nach einem der vorhergehenden, auf eine Markierungsvorrichtung (136) gerichteten Ansprüche, wobei die Strahlungsquelle (138) eine Vielzahl von modular aufgebauten Einzellichtquellen (144), insbesondere eine Vielzahl von modular aufgebauten Lichterzeugereinheiten (140) zur Erzeugung von ultraviolettem Licht, umfasst.

14. Markierungsvorrichtung (136) nach einem der vorhergehenden, auf eine Markierungsvorrichtung (136) gerichteten Ansprüche, wobei die Markierungsvorrichtung (136) mindestens eine Applikationsposition (152) umfasst, in welcher die Testelemente (112, 114, 116; 246, 254) mit der Fehlermarkierung versehen werden, wobei die Markierungsvorrichtung (136) weiterhin mindestens einen Wellenleiter (148), insbesondere einen Lichtwellenleiter, umfasst, um die Strahlung (156) von der Strahlungsquelle (138) zu der Applikationsposition (152) zu leiten.

15. Markierungsvorrichtung (136) nach dem vorhergehenden Anspruch, wobei der Wellenleiter (148) mindestens einen Faserlichtleiter, insbesondere einen Kunststofffaserlichtleiter (148), umfasst.

16. Markierungsvorrichtung (136) nach dem vorhergehenden Anspruch, wobei eine Vielzahl von Faserlichtleitern zu einem Faserbündel (146) zusammengefasst ist, wobei die Markierungsvonichtung (136) weiterhin mindestens einen Querschnittswandler (150) aufweist, wobei der Querschnittswandler (150) eingerichtet ist, um eine Vielzahl von Faserlichtleitern (148) mindestens eines Faserbündels (146) derart zu haltern, dass Faserenden (186) der Faserlichtleiter (148) in der Applikationsposition (152) in einem vorgegebenen Muster (188) angeordnet sind.

17. Markierungsvorrichtung (136) nach dem vorhergehenden Anspruch, wobei das Muster (188) der Faserenden (186) mindestens eines der folgenden Muster aufweist: ein Linienmuster; ein Matrixmuster, insbesondere ein Rechteckmatrixmuster.

18. Markierungsvorrichtung (136) nach einem der sechs vorhergehenden Ansprüche, wobei die Markierungsvorrichtung (136) in der Applikationsposition (152) einen Führungstisch (154) aufweist, wobei in dem Führungstisch (154) mindestens zwei, vorzugsweise mindestens fünf, Testelemente (112, 114, 116; 246, 254) gleichzeitig mit der Strahlung (156) beaufschlagbar sind.

19. Herstellungsvorrichtung (110) zur Herstellung von Testelementen (112, 114, 116; 246, 254), insbesondere zur Herstellung von Testelementen (112, 114, 116; 246, 254) nach einem der vorhergehenden, auf ein Herstellungsverfahren gerichteten Ansprüche, wobei die Testelemente (112, 114, 116; 246, 254) eingerichtet sind, um mindestens einen Analyten in einer Probe (220) nachzuweisen, wobei die Herstellungsvorrichtung (110) eine Produktionsvorrichtung (122) zur Herstellung einer Mehrzahl von Testelementen (112, 114, 116; 246, 254) aufweist, wobei die Herstellungsvorrichtung (110) weiterhin mindestens eine Markierungsvorrichtung (136) nach einem der vorhergehenden, auf eine Markierungsvorrichtung (136) gerichteten Ansprüche aufweist.

20. Herstellungsvorrichtung (110) nach dem vorhergehenden Anspruch, wobei die Herstellungsvorrichtung (110) weiterhin mindestens eine Sortiervorrichtung (160) zum Aussortieren von als fehlerhaft markierten Testelementen (112, 114, 116; 246, 254) aufweist.

21. Herstellungsvorrichtung (110) nach dem vorhergehenden Anspruch, wobei die Herstellungsvorrichtung weiterhin eine Schneide- und Splicingvorrichtung (167) aufweist.

22. Herstellungsvorrichtung (110) nach einem der drei vorhergehenden Ansprüche, wobei die Herstellungsvorrichtung (110) weiterhin mindestens eine Testvorrichtung (130) aufweist, wobei die Testvorrichtung (130) eingerichtet ist, um die Testelemente (112, 114, 116; 246, 254) einer Fehlerprüfung zu unterziehen.

23. Analytisches Testgerät (206) zum Nachweis mindestens eines Analyten in einer Probe (220), umfassend mindestens ein nach einem der vorhergehenden, auf ein Herstellungsverfahren gerichteten Ansprüche hergestelltes Testelement (112, 114, 116; 246, 254), wobei das analytische Testgerät (206) weiterhin mindestens eine Abfragevorrichtung (230) aufweist, wobei die mindestens eine Abfragevonichtung (230) ausgestaltet ist, um die Fehlermarkicrung des Testelements (112, 114, 116; 246, 254) abzufragen.

24. Analytisches Testgerät (206) nach dem vorhergehenden Anspruch, wobei das analytische Testgerät (206) eingerichtet ist, um bei Erkennung, dass ein Testelement (112, 114, 116; 246, 254) als fehlerhaft markiert ist, mindestens eine der folgenden Aktionen durchzuführen: eine Warnung wird an einen Benutzer des analytischen Testgerätes (206) ausgegeben; es wird ein Test mit dem markierten Testelement (112, 114, 116; 246, 254) verhindert; es wird ein neues Testelement (112, 114, 116; 246, 254) aus einer Mehrzahl von Testelementen (112, 114, 116; 246, 254) bereitgestellt.

## Claims

1. Marking method for marking test elements (112, 114, 116; 246, 254), the test elements (112, 114, 116; 246, 254) being set up to detect at least one analyte in a sample (220), at least some of the test elements (112, 114, 116; 246, 254) being provided with a fault marking, which contains information about the faultiness of the test elements (112, 114, 116; 246, 254), **characterized in that** the test elements (112, 114, 116; 246, 254) include at least one radiation-sensitive material (202), at least one radiation (156) being applied to the test elements (112, 114, 116; 246, 254), the radiation (156) being set up to cause a marking in the form of at least one optically detectable change in the radiation-sensitive material (202).

2. Marking method according to the preceding claim, wherein the radiation (156) includes an electromagnetic radiation, in particular an ultraviolet radiation, in particular a radiation in the wavelength range between 250 nm and 400 nm, preferably in the wavelength range from 350 nm to 380 nm.

3. Marking method according to one of the two preceding claims, wherein at least one of the following light sources is used to generate the radiation (156): an incandescent lamp; a gas discharge lamp; a laser; a light-emitting diode (176); a flash lamp.

4. Marking method according to one of the preceding claims, wherein the optically detectable change includes at least one of the following changes: a colour change; a change of a luminescence, in particular of a fluorescence; a change of a reflectivity.

5. Marking method according to one of the preceding claims, wherein the at least one radiation-sensitive material (202) includes at least one test material of the test elements (112, 114, 116; 246, 254), the at least one test material being set up to change at least one measurable property, in particular a measurable electrical and/or optical property, if the at least one analyte is present in the sample (220).

6. Marking method according to one of the preceding claims, wherein the at least one radiation-sensitive material (202) includes at least one material of at least one marking field which is different from a test material of the test elements (112, 114, 116; 246, 254).

7. Production method for producing test elements (112, 114, 116; 246, 254), the test elements (112, 114, 116; 246, 254) being set up to detect at least one analyte in a sample (220), the method including the following steps:
- a majority of the test elements (112, 114, 116; 246, 254) is produced;
- at least one of the test elements (112, 114, 116; 246, 254) is subjected to a fault test, wherein it is established whether the test element (112, 114, 116; 246, 254) is faulty;
- at least one of the test elements (112, 114, 116; 246, 254) is provided with a fault marking, which contains information about the faultiness of the test element (112, 114, 116; 246, 254), a marking method according to one of the preceding claims being used.

8. Production method according to the preceding claim, wherein in a sorting-out step, test elements (112, 114, 116; 246, 254) which are marked as faulty are sorted out.

9. Production method according to one of the two preceding claims, wherein multiple test elements (112, 114, 116; 246, 254) are produced as smallware, with a common carrier tape (118; 260).

10. Production method according to one of the preceding claims directed at a production method, wherein after the method step in which at least one of the test elements (112, 114, 116; 246, 254) is provided with the fault marking, a checking step, which checks whether the fault marking was correctly applied, is carried out.

11. Marking device (136) for marking faulty test elements (112, 114, 116; 246, 254), in particular for carrying out a marking method according to one of the preceding claims directed at a marking method, the test elements (112, 114, 116; 246, 254) being set up to detect at least one analyte in a sample (220), the marking device (136) being set up to provide at least some of the test elements (112, 114, 116; 246, 254) with a fault marking, which contains information about the faultiness of the test elements (112, 114, 116; 246, 254), **characterized in that** the test elements (112, 114, 116; 246, 254) include at least one radiation-sensitive material (202), the marking device (136) having at least one radiation source (138) to apply at least one radiation (156) to the test elements (112, 114, 116; 246, 254), the radiation (156) being set up to cause a marking in the form of at least one optically detectable change in the radiation-sensitive material (202).

12. Marking device (136) according to the preceding claim, also having at least one test device (130), the test device (130) being set up to subject at least one of the test elements (112, 114, 116; 246, 254) to a fault test, the marking device (136) being set up to decide whether the test element (112, 114, 116; 246, 254) is faulty.

13. Marking device (136) according to one of the preceding claims directed at a marking device (136), wherein the radiation source (138) includes multiple modularly structured individual light sources (144), in particular multiple modularly structured light generator units (140) for generating ultraviolet light.

14. Marking device (136) according to one of the preceding claims directed at a marking device (136), wherein the marking device (136) includes at least one application position (152), in which the test elements (112, 114, 116; 246, 254) are provided with the fault marking, the marking device (136) also including at least one waveguide (148), in particular an optical waveguide, to guide the radiation (156) from the radiation source (138) to the application position (152).

15. Marking device (136) according to the preceding claim, wherein the waveguide (148) includes at least one fibre light guide, in particular a plastic fibre light guide (148).

16. Marking device (136) according to the preceding claim, wherein multiple fibre light guides are combined into a fibre bundle (146), the marking device (136) also having at least one cross-section converter (150), the cross-section converter (150) being set up to support multiple fibre light guides (148) of at least one fibre bundle (146) in such a way that fibre ends (186) of the fibre light guides (148) in the application position (152) are arranged in a specified pattern (188).

17. Marking device (136) according to the preceding claim, wherein the pattern (188) of the fibre ends (186) has at least one of the following patterns: a linear pattern; a matrix pattern, in particular a rectangular matrix pattern.

18. Marking device (136) according to one of the six preceding claims, wherein the marking device (136) in the application position (152) has a guide plate (154), it being possible to apply the radiation (156) simultaneously to at least two, preferably at least five, test elements (112, 114, 116.; 246, 254) in the guide plate (154).

19. Production device (110) for production of test elements (112, 114, 116; 246, 254), in particular for production of test elements (112, 114, 116; 246, 254) according to one of the preceding claims directed at a production method, the test elements (112, 114, 116; 246, 254) being set up to detect at least one analyte in a sample (220), the production device (110) having a production device (122) for production of multiple test elements (112, 114, 116; 246, 254), the production device (110) also having at least one marking device (136) according to one of the preceding claims directed at a marking device (136).

20. Production device (110) according to the preceding claim, the production device (110) also having at least one sorting device (160) for sorting out test elements (112, 114, 116; 246, 254) marked as faulty.

21. Production device (110) according to the preceding claim, the production device also having a cutting and splicing device (167).

22. Production device (110) according to one of the three preceding claims, the production device also having at least one test device (130), the test device (130) being set up to subject the test elements (112, 114, 116; 246, 254) to a fault test.

23. Analytical test device (206) for detecting at least one analyte in a sample (220), including at least one test element (112, 114, 116; 246, 254) which is produced according to one of the preceding claims directed at a production method, the analytical test device (206) also having at least one interrogation device (230), the at least one interrogation device (230) being designed to interrogate the fault marking of the test element (112, 114, 116; 246, 254).

24. Analytical test device (206) according to the preceding claim, the analytical test device (206) being set up so that when it is detected that a test element (112, 114, 116; 246, 254) is marked as faulty, it carries out at least one of the following actions: a warning is output to a user of the analytical test device (206); a test with the marked test element (112, 114, 116; 246, 254) is prevented; a new test element (112, 114, 116; 246, 254) is provided out of multiple test elements (112, 114, 116; 246, 254).

## Revendications

1. Procédé de marquage destiné au marquage d'éléments de test (112, 114, 116 ; 246, 254), dans lequel les éléments de test (112, 114, 116 ; 246, 254) sont disposés afin de détecter au moins un analyte dans un échantillon (220), les éléments de test (112, 114, 116 ; 246, 254) étant au moins partiellement munis d'un marquage de défaut, qui contient une information sur la défectuosité des éléments de test (112, 114, 116 ; 246, 254), **caractérisé en ce que** les éléments de test (112, 114, 116 ; 246, 254) présentent au moins une matière sensible au rayonnement (202), au moins un rayonnement (156) étant incident sur les éléments de test (112, 114, 116 ; 246, 254), le rayonnement (156) étant disposé afin de provoquer un marquage sous forme d'au moins une modification optiquement détectable dans la matière sensible au rayonnement (202).

2. Procédé de marquage selon la revendication précédente, dans lequel le rayonnement (156) comprend un rayonnement électromagnétique, en particulier un rayonnement ultraviolet, en particulier un rayonnement dans la plage de longueurs d'onde comprises entre 250 nm et 400 nm, de préférence dans la plage de longueurs d'onde de 350 nm à 380 nm.

3. Procédé de marquage selon l'une quelconque des deux revendications précédentes, dans lequel au moins l'une des sources lumineuses suivantes est employée pour produire le rayonnement (156) : une lampe à incandescence ; une lampe à décharge gazeuse, un laser ; une diode lumineuse (176) ; une lampe éclair.

4. Procédé de marquage selon l'une quelconque des revendications précédentes, dans lequel la modification optiquement détectable comprend au moins l'une des modifications suivantes : une modification de couleur ; une modification d'une luminescence, en particulier d'une fluorescence ; une modification d'une réflectivité.

5. Procédé de marquage selon l'une quelconque des revendications précédentes, dans lequel ladite au moins une matière sensible (202) au rayonnement comprend au moins une matière de test des éléments de test (112, 114, 116 ; 246, 254), ladite au moins une matière de test étant disposée, lors de la présence dudit au moins un analyte dans l'échantillon (220), de façon à modifier au moins une propriété mesurable, en particulier une propriété électrique et/ou optique mesurable.

6. Procédé de marquage selon l'une quelconque des revendications précédentes, dans lequel ladite au moins une matière sensible (202) au rayonnement comprend au moins une matière d'au moins un champ de marquage différent d'une matière de test des éléments de test (112, 114, 116 ; 246, 254).

7. Procédé de préparation destiné à la préparation d'éléments de test (112, 114, 116 ; 246, 254), dans lequel les éléments de test (112, 114, 116 ; 246, 254) sont disposés afin de détecter au moins un analyte dans un échantillon (220), le procédé comprenant les étapes suivantes :
- une pluralité des éléments de test (112, 114, 116 ; 246, 254) est préparée ;
- au moins l'un des éléments de test (112, 114, 116 ; 246, 254) est soumis à un contrôle de défaut dans lequel il est constaté si l'élément de test (112, 114, 116 ; 246, 254) est défectueux ;
- au moins l'un des éléments de test (112, 114, 116 ; 246, 254) est muni d'un marquage de défaut, qui contient une information sur la défectuosité de l'élément de test (112, 114, 116 ; 246, 254), un procédé de marquage selon l'une quelconque des revendications précédentes étant employé.

8. Procédé de préparation selon la revendication précédente, dans lequel les éléments de test (112, 114, 116 ; 246, 254) marqués comme étant défectueux sont triés dans une étape de triage.

9. Procédé de préparation selon l'une quelconque des deux revendications précédentes, dans lequel plusieurs éléments de test (112, 114, 116 ; 246, 254) sont préparés sous forme de conditionnement en bande avec une bande porteuse commune (118 ; 260).

10. Procédé de préparation selon l'une quelconque des revendications précédentes visant un procédé de préparation, dans lequel après l'étape de procédé, dans laquelle au moins l'un des éléments de test (112, 114, 116 ; 246, 254) est muni d'un marquage de défaut, une étape de contrôle est réalisée, dans laquelle on contrôle si le marquage de défaut a été appliqué correctement.

11. Dispositif de marquage (136) destiné au marquage d'éléments de test défectueux (112, 114, 116 ; 246, 254), en particulier pour réaliser un procédé de marquage selon l'une quelconque des revendications précédentes visant un procédé de marquage, dans lequel les éléments de test (112, 114, 116 ; 246, 254) sont disposés afin de détecter un analyte dans un échantillon (220), le dispositif de marquage (136) étant disposé afin de munir les éléments de test (112, 114, 116 ; 246, 254) au moins partiellement d'un marquage de défaut, lequel contient une information sur la défectuosité des éléments de test (112, 114, 116 ; 246, 254), **caractérisé en ce que** les éléments de test (112, 114, 116 ; 246, 254) présentent au moins une matière sensible (202) au rayonnement, le dispositif de marquage (136) présentant au moins une source de rayonnement (138), afin de frapper les éléments de test (112, 114, 116 ; 246, 254) avec au moins un rayonnement (156), le rayonnement (156) étant disposé afin de provoquer un marquage sous forme d'au moins une modification optiquement détectable dans la matière sensible (202) au rayonnement.

12. Dispositif de marquage (136) selon la revendication précédente, présentant en outre au moins un dispositif de test (130), dans lequel le dispositif de test (130) est disposé afin de soumettre au moins l'un des éléments de test (112, 114, 116 ; 246, 254) à un contrôle de défaut, le dispositif de marquage (136) étant disposé afin de décider si l'élément de test (112, 114, 116 ; 246, 254) est défectueux.

13. Dispositif de marquage (136) selon l'une quelconque des revendications précédentes visant un dispositif de marquage (136), dans lequel la source de rayonnement (138) comprend une pluralité de sources lumineuses individuelles (144) de structure modulaire, en particulier une pluralité d'unités productrices de lumière (140) de structure modulaire pour produire de la lumière ultraviolette.

14. Dispositif de marquage (136) selon l'une quelconque des revendications précédentes visant un dispositif de marquage (136), dans lequel le dispositif de marquage (136) comprend au moins une position d'application (152), dans laquelle les éléments de test (112, 114, 116 ; 246, 254) sont munis d'un marquage de défaut, le dispositif de marquage (136) comprenant en outre au moins un guide d'onde (148), en particulier un guide d'onde lumineuse, afin de conduire le rayonnement (156) de la source de rayonnement (138) vers la position d'application (152).

15. Dispositif de marquage (136) selon la revendication précédente, dans lequel le guide d'onde (148) comprend au moins une fibre optique, en particulier une fibre optique en matière plastique (148).

16. Dispositif de marquage (136) selon la revendication précédente, dans lequel une pluralité de fibres optiques est réunie dans un faisceau de fibres (146), le dispositif de marquage (136) présentant en outre au moins un transformateur de section (150), le transformateur de section (150) étant disposé afin de maintenir une pluralité de fibres optiques (148) d'au moins un faisceau de fibres (146), de façon à ce que les extrémités de fibres (186) des fibres optiques (148) sont disposées dans la position d'application (152) selon un modèle prédéterminé (188).

17. Dispositif de marquage (136) selon la revendication précédente, dans lequel le modèle (188) des extrémités de fibres (186) présente au moins l'un des modèles suivants : un modèle linéaire ; un modèle matriciel, en particulier un modèle matriciel rectangulaire.

18. Dispositif de marquage (136) selon l'une quelconque des six revendications précédentes, dans lequel le dispositif de marquage (136) dans la position d'application (152) présente une table de guidage (154), le rayonnement (156) pouvant être simultanément incident sur au moins deux, de préférence au moins cinq éléments de test (112, 114, 116 ; 246, 254) dans la table de guidage (154).

19. Dispositif de préparation (110) destiné à la préparation d'éléments de test (112, 114, 116 ; 246, 254), en particulier à la préparation d'éléments de test (112, 114, 116 ; 246, 254) selon l'une quelconque des revendications précédentes visant un procédé de préparation, dans lequel les éléments de test (112, 114, 116 ; 246, 254) sont disposés afin de détecter au moins un analyte dans un échantillon (220), le dispositif de préparation (110) présentant un dispositif de production (122) pour préparer une pluralité d'éléments de test (112, 114, 116 ; 246, 254), le dispositif de préparation (110) présentant en outre au moins un dispositif de marquage (136) selon l'une quelconque des revendications précédentes visant un dispositif de marquage (136).

20. Dispositif de préparation (110) selon la revendication précédente, dans lequel le dispositif de préparation (110) présente en outre au moins un dispositif de triage (160) pour trier les éléments de test (112, 114, 116 ; 246, 254) marqués comme étant défectueux.

21. Dispositif de préparation (110) selon la revendication précédente, dans lequel le dispositif de préparation présente en outre un dispositif de coupe et d'épissurage (167).

22. Dispositif de préparation (110) selon l'une quelconque des trois revendications précédentes, dans lequel le dispositif de préparation (110) présente au moins un dispositif de test (130), le dispositif de test (130) étant disposé afin de soumettre les éléments de test (112, 114, 116 ; 246, 254) à un contrôle de défaut.

23. Appareil de test analytique (206) pour détecter au moins un analyte dans un échantillon (220), comprenant au moins un élément de test (112, 114, 116 ; 246, 254) préparé selon l'une quelconque des revendications précédentes visant un procédé de préparation, dans lequel l'appareil de test analytique (206) présente en outre au moins un dispositif de scrutation (230), ledit au moins un dispositif de scrutation (230) étant conçu de façon à scruter le marquage de défaut de l'élément de test (112, 114, 116 ; 246, 254).

24. Appareil de test analytique (206) selon la revendication précédente, dans lequel l'appareil de test analytique (206) est disposé afin d'exécuter au moins l'une des actions suivantes lors de la reconnaissance qu'un élément de test (112, 114, 116; 246, 254) est marqué comme étant défectueux : un avertissement est émis à l'intention d'un utilisateur de l'appareil de test analytique (206) ; un test avec l'élément de test marqué (112, 114, 116 ; 246, 254) est empêché ; un nouvel élément de test (112, 114, 116 ; 246, 254) est préparé à partir d'une pluralité d'éléments de test (112, 114, 116 ; 246, 254).
